# EUROPEAN PATENT SPECIFICATION

(11) **EP 0 571 593 B1**
(45) Date of publication and mention of the grant of the patent: **30.10.1996**
(21) Application number: 92924727.8
(22) Date of filing: 17.11.1992
(51) Int. Cl.: C07C 237/46, A61K 49/04

(54) **NEW NON IONIC IODIZED AGENTS FOR X-RAY CONTRASTING, METHOD FOR PREPARING THEM AND GALENICAL COMPOSITIONS CONTAINING THEM**
NICHTIONISCHE JODHALTIGE RÖNTGENSTRAHLENKONTRASTMITTEL, VERFAHREN ZU IHRER HERSTELLUNG UND DIESE ENTHALTENDE ZUSAMMENSETZUNGEN
NOUVEAUX AGENTS IODES NON IONIQUES DE CONTRASTE AUX RAYONS X, PROCEDE POUR LEUR PREPARATION ET COMPOSITIONS GALENIQUES LES CONTENANT

(30) Priority: 18.11.1991 WO PCT/ES91/00080
(43) Date of publication of application: 01.12.1993
(73) Proprietor: CENTRO INVESTIGACION JUSTESA IMAGEN S.A., E-28028 Madrid (ES)
(72) Inventor: MARTIN JIMENEZ, José L., E-28220 Majadahonda (ES); CARRETERO COLON, José M, E-28030 Madrid (ES); MARTINEZ SANZ, Antonio, E-28047 Madrid (ES); ALONSO SILVA, Ignacio, E-28017 Madrid (ES); HARTO MARTINEZ, Juan R., E-28010 Madrid (ES)
(74) Representative: Lehmann Novo, Maria Isabel
(86) International application number: ES9200075
(87) International publication number: WO9310079

(56) References cited:
- EP-A- 0 011 849
- EP-A- 0 049 745
- FR-A- 2 272 640
- US-A- 4 065 554

## Description

The present invention refers to new iodinated, non-ionic x-ray contrast media with a dimeric structure, pharmaceutical compositions which contain them, and a preferred method for preparing them.

These media are intended for contrasting the body structures and organs of the body by means of intravenous administration (specially in urography, and vascular and peripheral angiography), as also in other radiological techniques, such as lymphography and myelography, investigation of the valves of the heart, as also neuro-radiological techniques, intended to show up empty spaces consisting of cavities containing cephalorhachidian liquid, as also in bronchography, hysterosalpingography and arthrography.

### BACKGROUND

For the X-ray examination of, for example, the efferent urinary organs or the angiographic examination of blood vessels, very compatible salts of 2,4,6-triiodo-benzoic acids have been developed. However, these ionic compounds, when used at a high dosage, are not tolerated without side-effects, although their toxicity is generally low.

An adequate display of not only the vascular system and the efferent urinary system, but also of the cerebrospinal cavities and other systems, requires the use of high doses of the contrasting agent or highly concentrated solutions of the same. At the same time, the physical-chemical properties of contrasting agents become important, because they are responsible for serious pharmacological effects, such as pain, a lowering of blood pressure, injury of the blood vessels, and many other effects.

In neuroradiology, the empty spaces consist of various cavities which connect with the central nervous system. They include, for example, the ventricles of the brain, the cisterns, the sub-arachnoidal space around the brain, as also the spinal canal. The radiological examination of these cavities is usually subdivided into three main groups: ventriculography, cisternography and myelography.

The demands made on the physical -chemical properties of the contrasting agents, such as viscosity, which has been adapted to make them not only easier to use, but also to avoid premature emergence from the empty spaces it is wished to display, and the correct osmolality to reduce the overall effect of osmotic processes, are especially important. However, contrasting agents should not remain for too long in the spinal canal, which is the case with the iodinated oils which have been used up till now, and which are insoluble in water and metabolise slowly, Finally, as will readily be understood, the demands made regarding the compatibility of x-ray contrasting agents are specially high in neuroradiology. The amounts of X-ray contrasting agents are large in comparison with the amount of liquid in the space being examined.

### SUMMARY OF THE INVENTION

To avoid these drawbacks, non-ionic, water-soluble contrasting agents have 'been developed, which present a series of advantages:

They have a lower osmotic pressure and, therefore, cause less pain and less damage to the endothelium. Moreover, their concentration in the urine is higher and, in the case of a sub-arachnoidal injection, arachnoiditis rarely occurs.

In the case of myelography, when non-ionic contrasting agents are used, there is only a slight tendency towards convulsive states (epilepto-genicity) observed. However, with the METRIZAMIDE (2-(3-acetamido-2,4,6-triiodo-5-[N-methylacetamido]-benzamido)-2-deoxi-D-glucose) or IOPAMIDOL (bis[1,3-dihydroxy-propylamide] of 5-α-hydroxypropionyl-amino-2,4,6-triiodo-isophthalic acid), it has not been possible, in angiography and myelography applications, to prepare sufficiently concentrated and X-ray-opaque solutions, which were not also hypertonic in relation to blood and to the cephalorhachidian fluid.

These agents, in spite of being strongly hydrophilic and of low osmotic pressure, are highly viscous or barely soluble and have certain toxic effects.

As a patent which describes important non-ionic agents like the ones just mentioned, and which are also dimers, there is the Spanish Patent 381.202, which refers to compounds with the general formula: 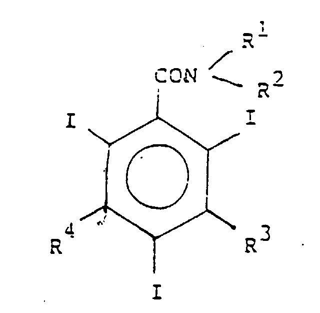 where R¹ and R² can be identical or different, and can be hydrogen atoms or alkyl, hydroxyalkyl or acyloxialkyl groups, and R³ and R⁴ can also be identical or different, are hydrogen atoms, acylamino groups with the formula NR⁵AC where R⁵ is a hydrogen atom or an alkyl, hydroxyalkyl or acyloxialkyl group or an acyl group and Ac is the acyl group; acylaminomethyl groups with the formula CH₂NR⁵Ac; or carbamoyl groups with the formula CONR⁶R⁷ in which R⁶ and R⁷ have the same character as R⁵.

In the publication of European Patent EP-A1-002992, non-ionic compounds with the following general formula are described: 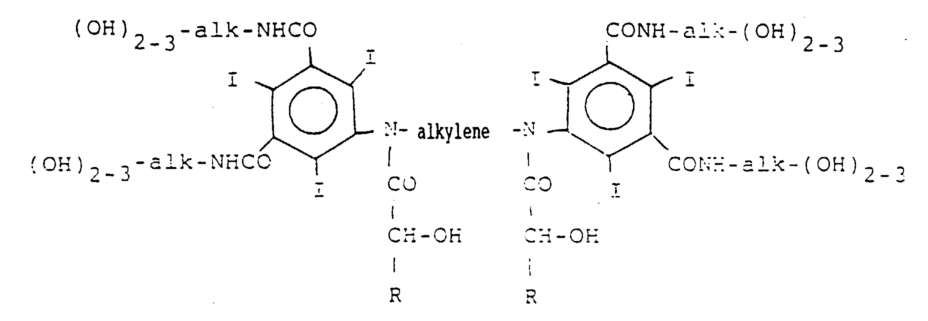 where,
- (OH)₂₋₃-alk: represents 1,3-dihydroxy-isopropyl, 2,3-dihydroxy-propyl or 1,3-dihydroxymethylisopropyl;
- R: represents a hydrogen or methyl; and
- "alkylene": represents a divalent alkylene radical of 2 to 10 carbon atoms, which may be replaced by hydroxy functions, or a mono-, di-, or polyoxaalkylene radical of 4 to 12 atoms of carbon which may be substituted with hydroxy functions, these being intended as compounds for vasography, urography, bronchiography and to reveal body cavities and places where body liquids accumulate, by being opaque compounds. It is pointed out that these compounds are highly soluble in water, have the highest compatibility and slight osmolality.

In the publication of European Patent EP-A1 0,049.745 which is the equivalent of Spanish Patent 460.007, non-ionic compounds with the following general formula are described: 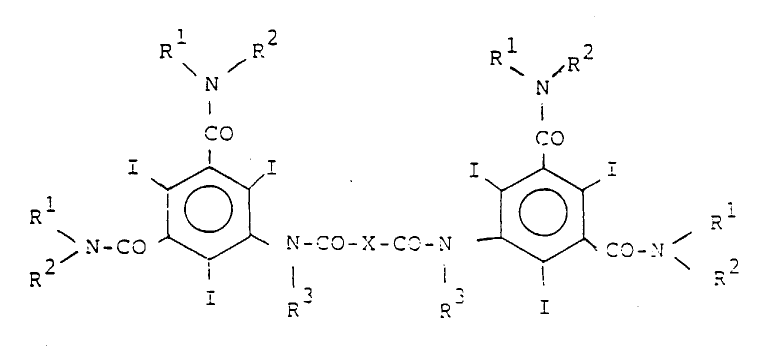 where,
- R¹: represents a lower mono- or poly-hydroxyalkyl radical with a linear or branched chain;
- R²: represents a hydrogen atom, a lower alkyl radical or R¹;
- R³: represents a lower mono- or di-hydroxyalkyl radical; and
- X: represents a direct bond or an alkylene linear or branched chain which may be interrupted by one or various oxygen atoms and replaced with hydroxy or alkoxy groups.

A compound which is included in this general formula which has been mentioned especially is that known under the name of IOTROLAN, with the formula N,N'-dimethyl-N,N'-bis[3,5-(l-hydroxymethyl-2,3-dihydroxy-propyl-carbamoyl) -2,4,6-triiodo-phenyl]-diamide of malonic acid, in which therefore:
X = methylene, R¹ = R² = 1-hydroxymethyl-2,3-dihydroxy-propyl and R³ = methyl.

It is pointed out that these compounds are suitable for providing opacity as X-ray contrasting agents.

In the publication of European Patent EP-A1-108.638 non-ionic compounds with the following general formula are described: 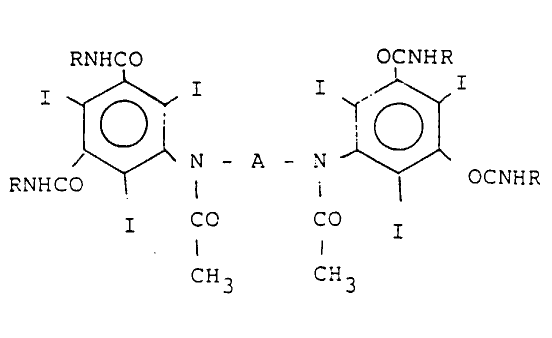 where
- R is a: -CH(CH₂OH)₂[1,3,dihydroxy-2-propyl], -CH₂CH(OH)CH₂OH[2,3-dihydroxypropyl] group; and
- A is a: -CH₂CH(OH)CH₂-[2-hydroxypropane-1,3-diyl] or -CH₂CH(OH)CH(OH)CH₂-[2,3-dihydroxy-butane-1,4-diyl],
which are indicated as having good opacity characteristics as contrast media for x-rays.

Finally, in the document of European Patent 33.426, compounds with the following general formula A [and B] are described: 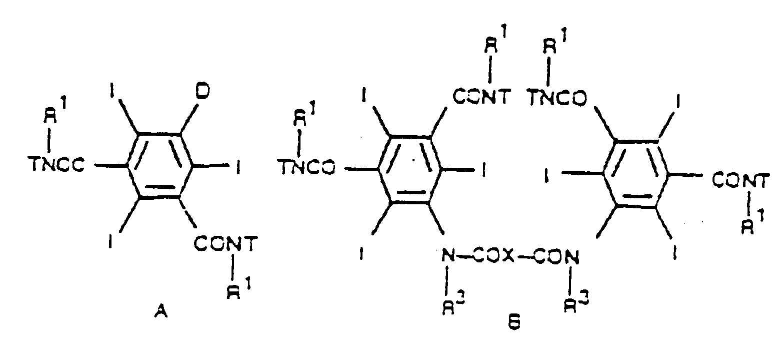 where,
- D is: 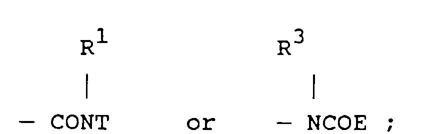
- T is: 1,3,4-trihydroxybut-2-yl;
- R¹ is: hydrogen, methyl or ethyl;
- E is: C₁₋₃ alkyl, usually C₁₋₂ alkyl, which may be substituted with one or two OH, alkoxy₁₋₃ or C₁₋₃ groups; and
- X is: a simple or C₁₋₂ alkylene group, preferably C₁₋₂ which containsfrom nought to two C₁₋₃ hydroxy and/or alkoxy groups, each bridge carbon atom having a maximum of one substitute.

To prepare these compounds, dioxolane-type protector groups are used for the vicinal OH groups.

Included in these compounds the aforementioned IOTROLAN is to be found.

The authors of the present invention have discovered a new group of non-ionic dimeric compounds derived from dicarbamoyl-triiodoisophthalic acid which incorporate a glycine bridge in their molecule, and have the general formula I: 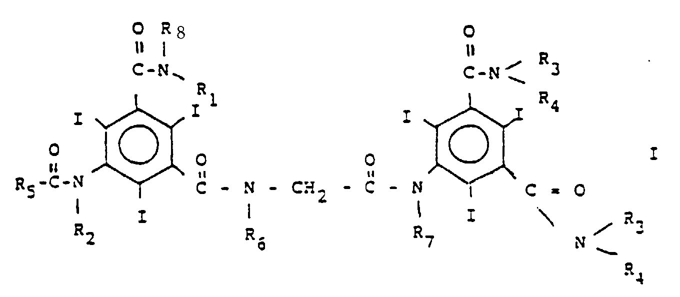 where,
- R₁: represents a linear or branched C₂-C₄ polyhydroxylalkyl radical;
- R₂: represents hydrogen, methyl, 2-hydroxyethyl, a linear C₂-C₃ polyhydroxyalkyl radical, 2-methoxyethyl or 2-hydroxy-3-methoxypropyl;
- R₃ and R₈: are the same or different, and represent hydrogen, methyl or 2-hydroxyethyl;
- R₄: represents a linear or branched C₂ to C₄ polyhydroxyalkyl radical;
- R₅: represents methyl, hydroxymethyl or methoxymethyl;
- R₆ and R₇: are the same or different, and represent hydrogen, methyl, 2-hydroxyethyl 2-methoxyethyl, 2,3-dihydroxypropyl or 2-hydroxy-3-methoxypropyl.
- R₂ and R₅: together form a group of the type 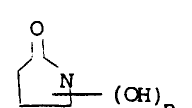 where n can be 0 to 3.

It has been shown that these compounds have a higher iodine content and that their aqueous solutions have lower viscosities to those compounds previously indicated, as also only slight toxicity, higher solubility in water and, above all, they are very economical to synthesise and purify.

The very low osmolality is achieved because of the reduction of the number of dissolved particles. Owing to this reduction in osmolality, a significant reduction in side-effects due to tissue damage, deformation of erythrocytes and vasodilatation can be achieved, it being possible to make this reduction larger in the products of this invention when the ratio of iodine atoms to total particles dissolved is 6:1.

In addition, it has been observed that the compounds of the present invention have a pronounced effect on the intra-molecular aggregation resulting in actual osmolalities of the solutions which are even lower than the theoretical one, resulting, in those cases examined, in solutions which are hypotonic with respect to blood or to the cephalorhachidian fluid.

Another important aspect, which has been already quoted, is the viscosity of aqueous solutions. In general, this parameter increases with the number of OH groups present in the molecule, but as these groups are essential to achieve the necessary hydrosolubility of the compounds, in most cases it is necessary to make a compromise; in some cases, the substitution of an OH group by an -OCH₃ group has even resulted in compounds which are very soluble without a substantial increase in viscosity.

It has been shown that the compounds investigated present a series of isomeric forms due to the existence of chiral carbon atoms (optical isomers), as also the presence of restricted rotation of the N-CO bonds due to the proximity of the bulky iodine atoms (isomers E and Z), the invention extending also to the isomeric forms of these compounds, including the optically active, racemic and "meso" forms.

The solubility of the compounds in water at 20°C easily reaches values of 400 mg of I/ml apparently without a tendency to crystallise due to supersaturation. It is assumed that there a relationship between the solubility in water with the large number chemically isomeric species.

Similarly, their toxicity, measured as the lethal dose at 50%, LD₅₀, is much reduced as has been shown by the studies carried out.

The invention also extends to galenic compositions which include compounds with the general formula I, at concentrations between 100-400 mg of I/ml, and incorporate pH stabilisers, such as the buffer Tris (=tris(hydroxymethyl)aminomethane and its phosphate, citrates and bicarbonate salts, as well as sterile andapyrogenous water. The formulation may also incorporate complexing agents for heavy metals, for example, the sodium and/or calcium salts of ethylenediaminetetracetic acid, and other chelating agents which are pharmaceutically acceptable.

It is evident that these compositions, because of the higher proportion of iodine contained in compounds of Formula I, that for a given concentration of iodine per millilitre, less quantity of active substance is required, resulting in an economy in the preparation of these compositions.

From these compounds, those preferred are those from Examples 1 - 38, whose critical properties (solubility, viscosity, osmolality and iodine concentration as a percentage) have been found to be very advantageous. To facilitate identification, they are summarised in the following table, Table I, where the equivalence of the symbols R₁ to R₈, and the value of the aforesaid properties are given.

As particularly of interest, the compounds of Examples 9, 11 and 36 stand out.

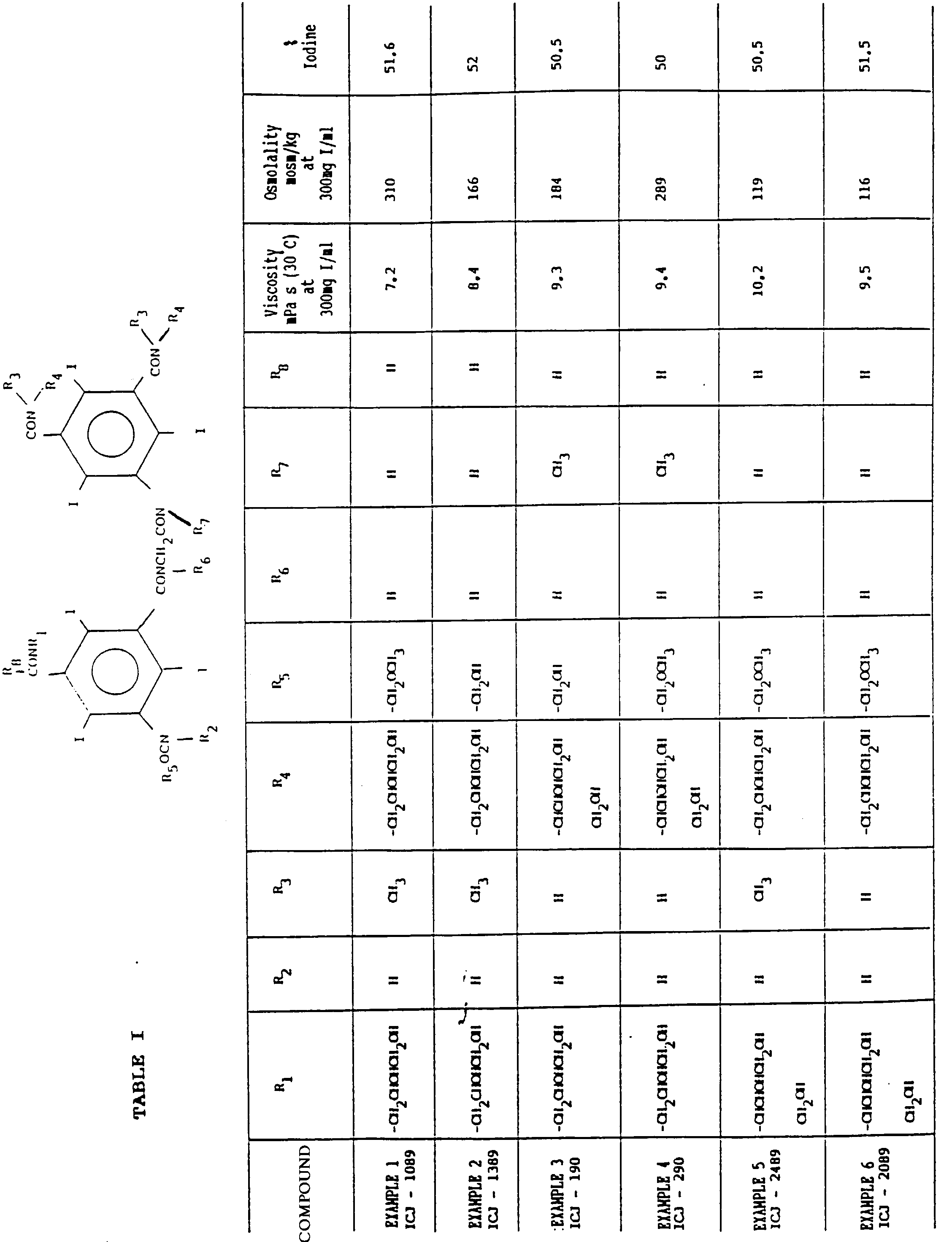

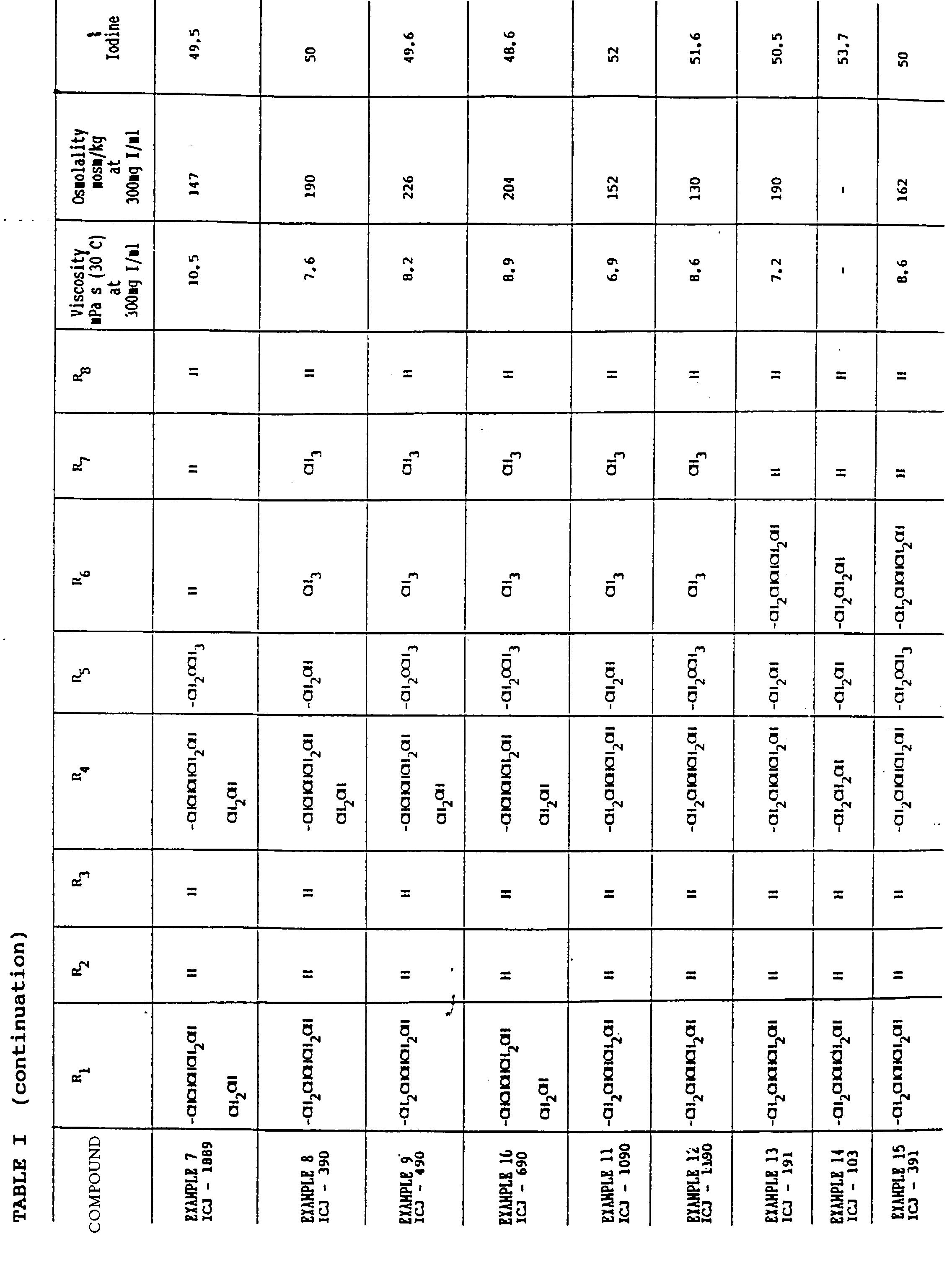

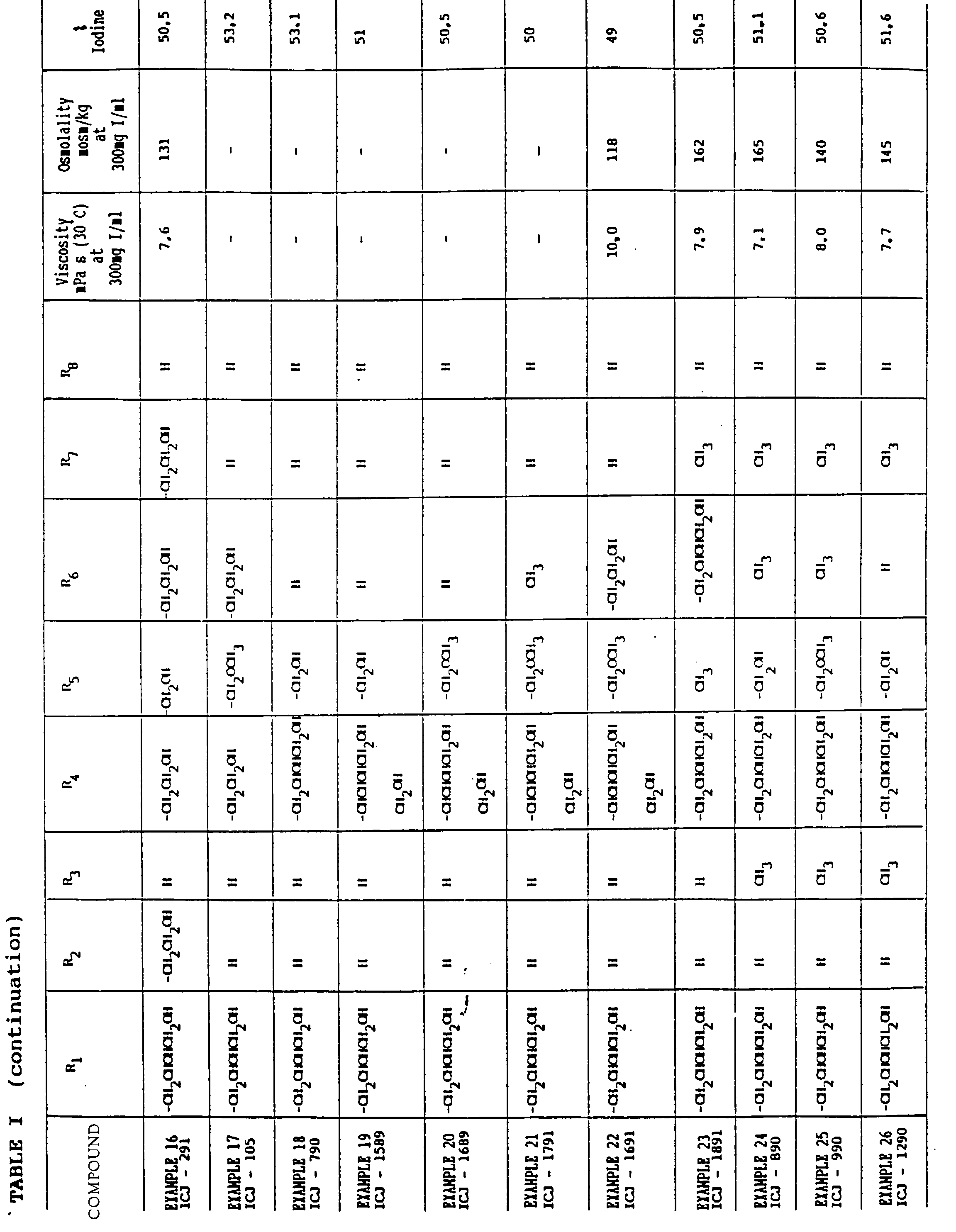

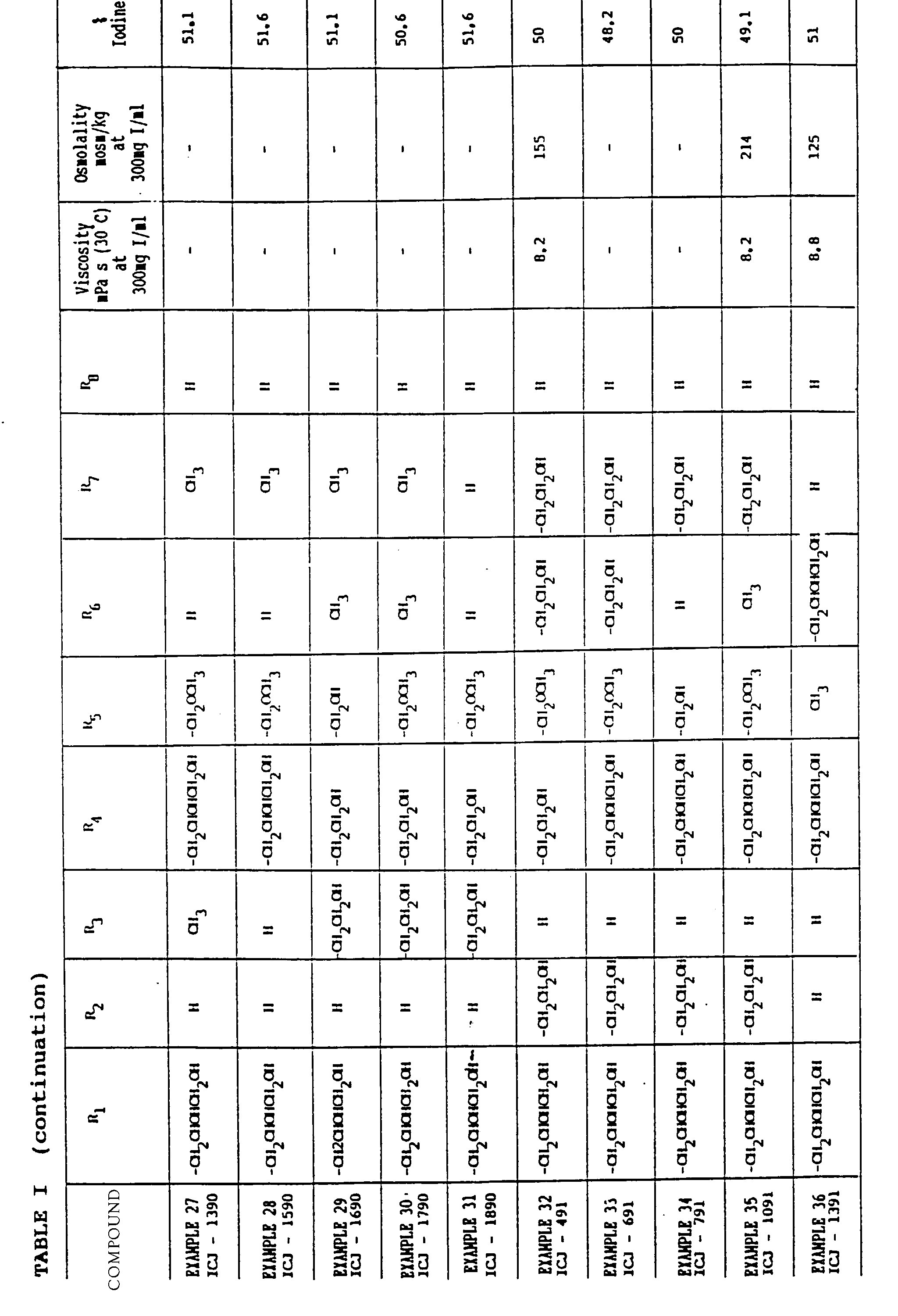

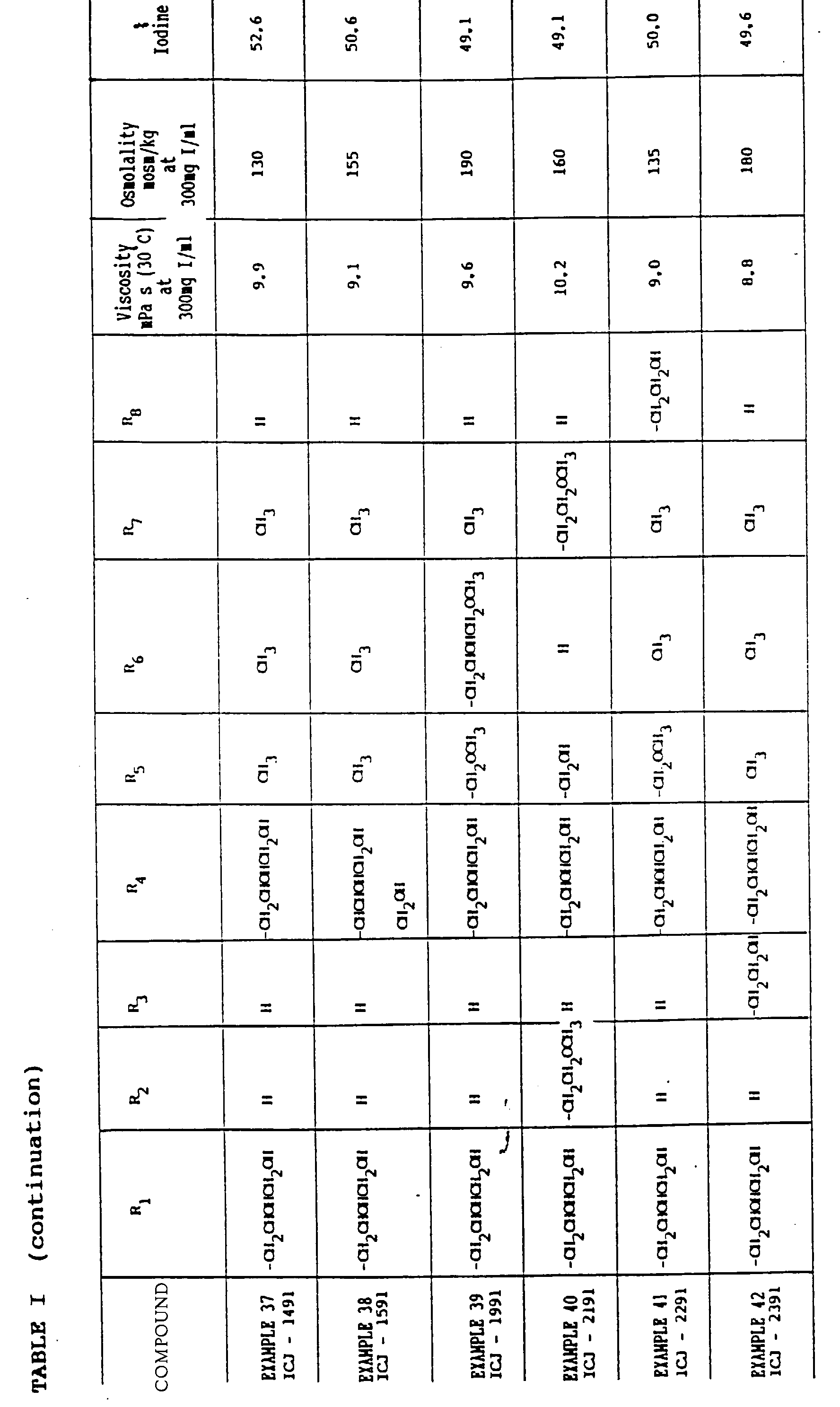

To demonstrate the reduced intravenous toxicity, Sprague-Dawley rats of both sexes with a weight between 90 and 120 g were used, and which were administered the compounds via the caudal vein at a constant rate of 2.2ml/min (Salvesen S. Acta Radiologica, Suppl. 362, page 73, 1980). After the injection, the animals were observed to record their immediate reactions and the respective mortalities were recorded during a period of seven days, followed by calculation of the lethal dose for 50% of the animals, LD₅₀.

The most preferred compounds were studied, from Examples 9, 11 and 36, and they were compared with IOTROLAN as a reference which has already been mentioned. The physical-chemical and toxicological data appear in the following table:

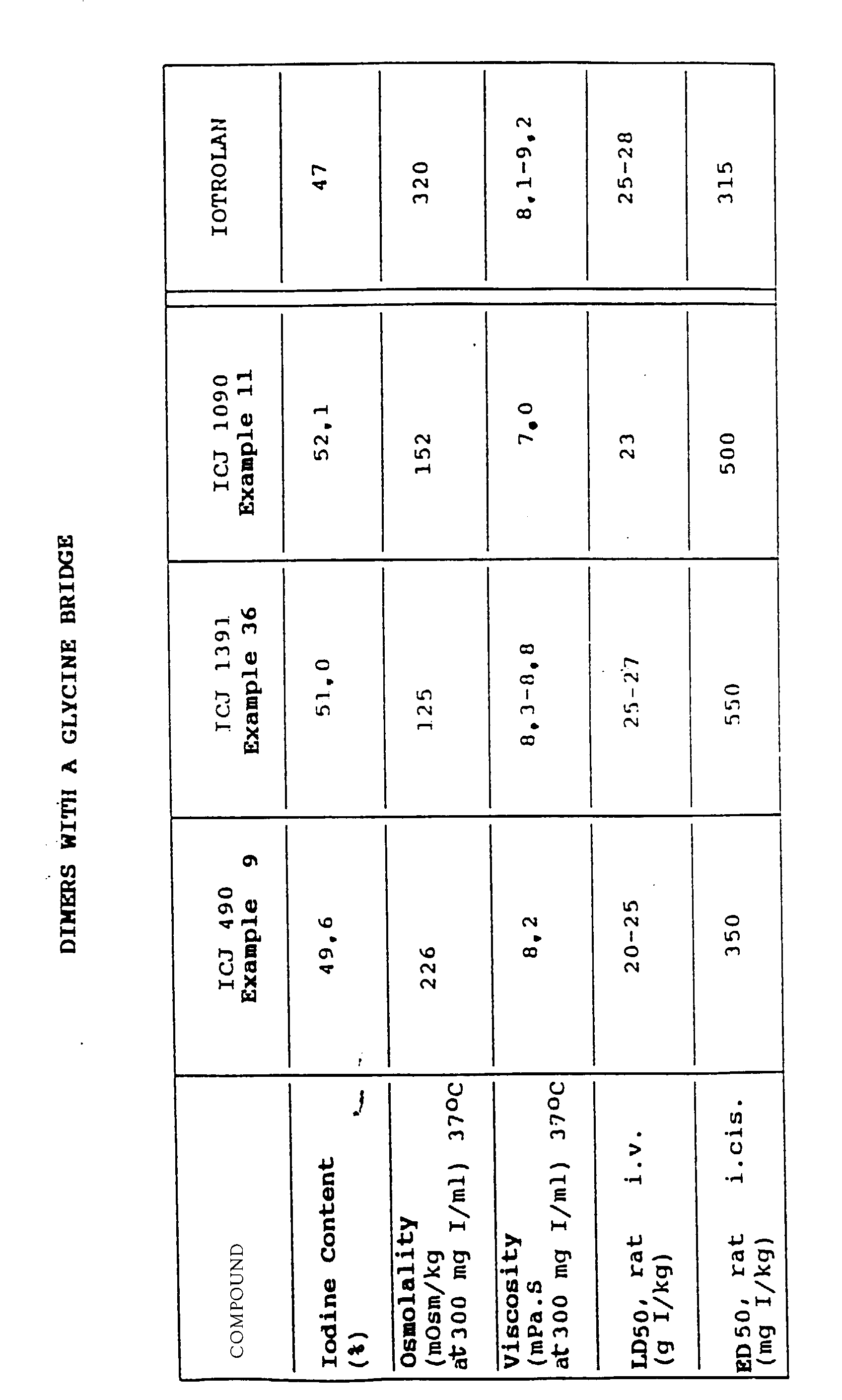

According to a preferred method, the compounds of formula I are prepared by reacting a compound of formula II 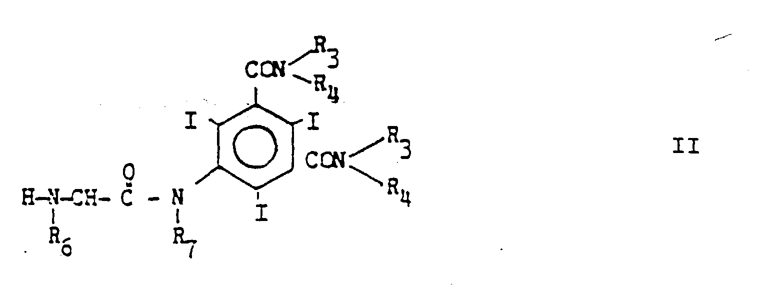 with an acid chloride with the formula III 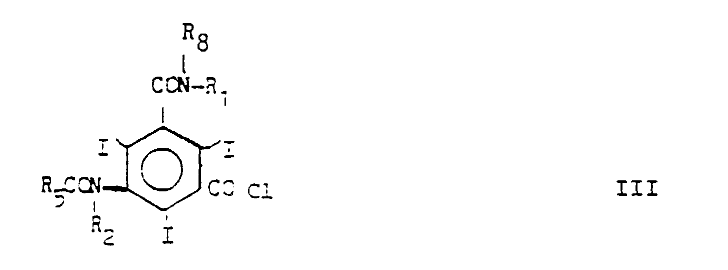 in the presence of a basic catalyst, the radicals R₁ to R₈ having the significance given previously.

During the reaction, the OH groups are protected as acetates or, when the OH groups are vicinal, as dioxepane. These protective groups are easily eliminated by conventional methods used in chemistry, such as hydrolysis.

The final conpounds of general formula I are purified by treatment with ion-exchange resins, by conventional purification methods, or by means of preparative chromatography.

There follows a description of the preparation of the preferred compounds mentioned in Table I.

### Example 1

### N,N'-bis-(2,3-dihydroxypropyl)-N,N'-dimethyl-5-{3-[N-(2,3-dihydroxypropyl)carbamoyl]-5-methoxyacetamido-2,4,6-triiodo benzoylaminoacetamido}-2,4,6-triiodoisophthalamide (lCJ-1089)

### a) N-(2,3 diacetoxypropyl)-5-methoxyacetamido-2,4,6-triiodoisophthalamoyl chloride.

### Method I

To a solution of N-(2,3-diacetoxy-propyl)-5-amino-2,4,6-triiodoisophthalamoyl chloride (22.03g, 30 mmcl) in DMA (80 ml) cooled to 0-5°C, is slowly added a solution of methoxyacetyl chloride (8 g, 90 mmol) in DMA (20 ml). Once the addition has been completed, it is stirred at room temperature during the night. The reaction medium is distributed between ethyl acetate (350ml) and water (200 ml).

The aqueous phase is extracted with ethyl acetate (3 x 50 ml) the organic extracts are combined and washed with a 5% sodium bicarbonate solution(2 x 50 ml) and a saturated solution of sodium chloride (100ml). The product is dried over anhydrous MgSO₄ and the solvent is removed at reduced pressure. The product thus obtained is purified by recrystallisation from ethyl acetate (90 ml).
M.p.: 211/213°C. Yield: 62%

### Method II

A mixture of methoxyacetic acid (39.6 g, 0.44 mol) and thionyl chloride (30.5 ml, 0.42 mol). is heated at 50°C for two hours. It is cooled to room temperature and it is slowly added to a solution cooled to 0-5°C of N-(2,3-diacetoxypropyl)-5-amino-2,4,6-triiodoisophthalamic acid (78.8 g, 0.11mol) in DMA (300 ml). Once the addition is concluded, it is stirred at room temperature until fully converted into N-(2,3-diacetoxypropyl)-5-methoxyacetamido-2,4,6-triiodoisophthalamic acid (TLC with AcOEt/CHCl₃/AcOH 6:2:1) which is not isolated.

It is cooled again to 0-5°C and thionyl chloride (32 ml, 0.44 mol) is slowly added, and stirring is maintained for 30 minutes. The reaction medium is distributed between ethyl acetate (1120ml) and water (1060 ml). The aqueous phase is extracted with ethyl acetate (3 x 250ml) the organic extracts are combined and washed with sodium bicarbonate at 10% (3 x 200 ml) and a saturated solution of sodium chloride (1 x 250ml). It is dried over anhydrous MgSO₄ and the solvent is removed at reduced pressure. The product thus obtained is purified by recrystallisation from ethyl acetate.
Yield: 75%.

### b) N,N'-bis-(2,3-dihydroxypropyl)-N,N'-dimethyl-5-{3-[N-(2-3-dihydroxypropyl)carbamoyl]-5-methoxyacetamido-2,4,6-triiodo benzoylaminoacetamido}-2,4,6-triiodoisophthalamide.

To a suspension of N,N'-bis-(2,3-dihydroxy-propyl)-N,N'-dimethyl-5-aminoacetamido-2,4,6-triiodoisophthalamide ⁽³⁾ (12 g, 0.015 mol) y and sodium carbonate decahydrate (13.lg, 0.046 mol) in DMA (60 ml), is added, with vigorous stirring, the compound previously described in example 1a) (13.5 g, 0.017 mol). It is stirred continuously at room temperature until the next day. It is filtered, and the filtrate is concentrated to dryness at reduced pressure. The residue is dissolved in methanol/water (1:1) and is hydrolysed with 50% NaOH (pH 11.5) at 40°C. It is neutralised with concentrated hydrochloric acid (pH 6.5) and evaporated to dryness at reduced pressure. The residue is dissolved in methanol and precipitated from isopropanol. Filter and dry in vacuo.
Yield: 70%.
The product obtained is purified by preparative liquid chromatography.
M.p.: 200°C (sinters)/ 230-257°C (decomposes).

### Example 2

### N.N'-bis-(2,3-dihydroxypropyl)-N,N'-dimethyl-5-{3-[N-(2,3-dihydroxypropyl)carbamoyl]-5-hydroxyacetamido-2,4,6-triiodo benzoylaminoacetamido}-2,4,6-triiodoisophthalamide (lCJ-1389)

### a) N-(2,3-diacetoxypropyl)-5-acetoxyacetamido-2,4,6-triiodoisophthalamoyl chloride

### Method I

To a solution of N-(2,3-diacetoxy-propyl)-5-amino-2,4,6-tri-iodoisophthalamoyl chloride (66.lg, 0.090 mol) in DMA (260 ml) cooled to 0-5°C, is slowly added acetoxyacetyl chloride (36.9g, 0.270 mol). Stirring is maintained at room temperature until the next day. The reaction medium is distributed between ethyl acetate 400ml and water (800ml). It is extracted from the aqueous phase with ethyl acetate (3 x 250ml) and the combined organic extracts are washed with a 10% solution of sodium bicarbonate (2 x 200ml) and 10% sodium chloride (200ml). It is dried over anhydrous sodium sulphate and the solvent is removed under reduced pressure. The product obtained is cold recrystallised from chloroform. Yield: 88-90% M.p.: 202-204°C

### Method II

To a solution of N-(2,3-diacetoxypropyl)-5-amino-2,4,6-triiodoisophthalamic acid (65.6g, 0.092 mol) in DMA (250 ml) cooled to 0-5°C, is slowly added acetoxyacetyl chloride (50g, 0.366 mol). Stirring is maintained at room temperature for 3-4h., until it is totally transformed into N-(2,3-diacetoxypropyl)-5-acetoxyacetamido-2,4, 6-triiodoisophthalamic acid, which is not isolated.

It is cooled to 5-10°C, and thionyl chloride (26.5 ml, 0.366 mol) is slowly added and maintained at this temperature for 45 minutes.

The product is isolated from the reaction medium in the same manner as in Method 1. Yield: 75%.

b) N,N'-bis-(2,3-dihydroxypropyl)-N,N'-dimethyl-5-{3-[N-(2,3-dihydroxypropyl)carbamoyl]-5-hydroxyacetamido-2,4,6-triiodobenzoylaminoacetamido)-2,4,6-triiodoisophthalamide.

Following the general procedure given in example 1b) and starting from N,N'-bis(2,3-dihydroxy-propyl)-N,N'-dimethyl-5-aminoacetamido-2,4,6-triiodoisophthalamide (3) (20 g, 0.025 mol) and the compound described in example 2 a) (23.2 g, 0.028 mol). Gross yield: 70%. m.p.. 261-274°C (decomposes)

### Example 3

### N,N'-bis-(1-hydroxymethyl-2,3-dihydroxyprogyl)-5-{3[N-(2,3-dihydroxypropyl)carbamoyl]-5-hydroxyacetamido-2,4,6-triiodo benzoylaminoacetyl-N-methylamino}-2,4,6 -triiodoisophthalamide. (lCJ-190)

### a) 5-chloroacetyl-N-methylamino-2,4,6-triiodoisochthaloyl chloride.

60 g (104.74 mmol) of 5-N-methylamino-2,4,6-triiodo isophthalic acid is suspended in 400 ml of ethyl acetate and 51.8 g (435.74 mmoles) of thionyl chloride are added. It is heated to maintain reflux for 4 hours. A solution of dichloride is obtained. Thin-layer chromatography is carried using toluene-methanol (4:1) as an eluent. The product with an Rf=0.90 is observed. It is cooled to 60°C and 35.5 g (314.33 mmoles) chloroacetyl chloride is slowly added. It is, returned to reflux temperature and heated for 7 hours. After 1 hour, the desired product starts to crystallise.

Thin-layer chromatography is carried out using toluene-methanol (4:1) as an eluent, and the product with an Rf= 0.78 is observed.
The ethyl acetate is partially distilled off.
The product is cooled to room temperature and filtered off. Obtained: 61g (molar yield: 84%).

### b) N,N'-bis-(7-hydroxy-4,4-dimethyl-3,5-dioxepanyl)-5-chloroacetyl-N-methylamino-2,4,6-triiodoisophthalamide.

185 g (269.59 mmol) of 5-chloroacetyl-N-methylamino-2,4,6-triiodoisophthaloyl chloride are suspended in 555 ml of dioxane. 169.7 g of sodium carbonate decahydrate (593.10 mmol) and 95.6 g of 2,2-di-methyl-5-hydroxy-6-amino-1,3 dioxepane (593.10 mmol) are added and heated to 50°C. After 8 hours total conversion to diamide occurs. Thin-layer chromatography is carried out using acetone-ethyl acetate (1:1) as an eluent, and a product with an Rf of 0.70 is observed. Salts are filtered off and the solvent is evaporated to dryness.
Obtained: 226 g (Molar yield: 90%)

### c) N,N'-bis-(1-hydroxymethyl-2,3-dihydroxypropyl)-5-chloroacetyl-N-methylamino-2,4,6-triiodoisochthalamide.

100 g of the compound from example 3b) are taken and dissolved in 300 ml of methanol. 35% hydrochloric acid is added until a pH=1 is reached, and the mixture is heated at 40°C until total hydrolysis is achieved with a quantitative yield, The product is evaporated to dryness.
Obtained: 91,4 g (quantitative).

Thin-layer chromatography under the same conditions as in example 3b) retains the product totally at the origen. Using chloroform-methanol (3:2) as an eluent results in an Rf =0.57.

### d) N,N'-bis-(1-hydroxymethyl-2,3-dihydroxypropyl)-5-aminoacetyl-N-methylamino-2,4,6-triiodoisophthalamide.

23.6 g of the compound of example 3c) (27.58 mmol) are dissolved in 275 ml of aqueous ammonia, and then heated at 70°C for 24 hours. The product is evaporated to dryness. The product is dissolved in methanol, the hydrochloride is formed and the solvent is removed.

Thin-layer chromatography using chloroform -methanol (3:2) as a eluent produces a stain at the origen. Using methanol as an eluent it produces a stain retained at Rf=0.65. Obtained: 12.5 g (molar yield: 54%)

### e) N,N'-bis-(1-hydroxymethyl-2,3-dihydroxypropyl)-5-{3-[N-(2,3-dihydroxypropyl)carbamoyl]-5-hydroxyacetamido-2,4,6 -triiodobenzoylaminoacetyl-N-methylamino}-2,4,6-triiodo isophtalamide.

14 g (16.74 mmol) of the compound from example 3d) is suspended in 60 ml of dimethylformamide, and 9.58g (33.48 mmol) of sodium carbonate are added. The product is stirred for 5 min. and 15.1 g (18.08 mmol) of the product from example 2a) are added. It is stirred at room temperature until the reaction is complete (24 hours).

The salts are filtered off, and the solvent which contains the dimer is evaporated to dryness. It is dissolved in 100 ml of methanol. It is then filtered and precipitated with 600 ml of isopropanol. It is filtered, drained and dried. Crude product: 26 g (Yield: 95%).

The acetate groups are hydrolysed and the substance is purified by preparative liquid chromatography.
Pure product: 12 g (molar yield: 48%).

### Example 4

### N,N'-bis-(1-hydroxymethyl-2,3-dihydroxypropyl)-5-{3-(N-(2,3-dihydroxypropyl)carbamoyl]-5-methoxyacetamido -2,4,6-triiodobenzoylaminoacetyl-N-methylamino}-2,4,6-triiodo isophthalamide. (lCJ- 290)

15.2 g (18.18mmcl) of the compound from example 3d) is suspended in 70 ml of DMF, and 10.38 g (36.28 mmol) of sodium carbonate decahydrate is added. The mixture is stirred for 5 min. and 15.8 g (19.59 mmol) of the compound from example 1a) is added. It is stirred at room temperature until the reaction is complete. (28 hours).

The salts are filtered off and the solvent is evaporated. The product is then dissolved in 100 ml of methanol organic salts are again filtered off) and it is precipitated in 600 ml of isopropanol. It is filtered off and dried. Crude product: 27 g (Yield: 92%).
The acetate groups are hydrolysed and the product is purified by preparative liquid chromatography.
Pure product: 13 g (molar yield: 47%).

### Example 5

### N,N'-bis-(2,3-dihydroxypropyl)-N,N'-dimethyl-5-{3-[N-(1-hydroxymethyl-2,3-dihydroxypropyl)carbamoyl]-5-methoxyace tamido -2,4,6-triiodobenzoylaminoacetamido}-2,4,6-triiodoisophthalamide. (lCJ -2489)

### a) N-(4,4-dimethyl-7-hydroxy- 3,5-dioxepanyl)-5-methoxyacetamido-2,4,6-triiodoisophthalamoyl chloride

To a cold solution of 5-methoxyacetamido-2,4,6-triiodoisophthaloyl chloride (30.05 g, 45 mmol) in DMA (150 ml), a solution of 6-amino-2,2-dimethyl-1,3-dioxepane-5-ol ⁽²⁾ (7.98g 49.5 mmol) and triethylamine (4.55 g, 45 mmol) in DMA (30 ml) is slowly added. It is stirred for 2 hours and the reaction medium is distributed between ethyl acetate (400 ml) and water (500 ml). The aqueous phase is extracted with ethyl acetate (2 x 50ml) and the combined organic extracts are washed with a 5% solution of sodium bicarbonate (50ml) and saturated sodium chloride (50 ml). It is then dried over anhydrous magnesium sulphate and dried under reduced pressure. The product thus obtained is dried in vacuo. Yield: 67% m.p.: 230°c (decomposes).

b ) N,N'-bis-(2,3-dihydroxypropyl)-N,N'-dimethyl-5-{3-[N-(1-hydroxymethyl-2,3-dihydroxypropyl)carbamoyl]-5-methoxyacetamido -2,4,6-triiodobenzoylaminoacetamido}-2,4,6-triiodoisophthalamide.

Following the general procedure described in example 1b) and starting from N,N'-bis(2,3-dihydroxy-propyl)-N,N'-dimethyl-5-aminoacetamido -2,4,6-triiodoisophthalamide (20 g, 0.025 mol) and the compound described in example 5a) (22.1 g, 0.088 mol), a partially-protected compound is obtained (Yield = 73%) which is treated with hydrochloric acid diluted in methanol. The resulting product is purified by preparative liquid cromatography.
M.p.: 263-275°C. (decomposes)

### Example 6

### N,N'-bis-(2,3-dihydroxypropyl)-5-{3-[N-(1-hydroxymethyl-2,3-dihydroxypropyl)carbamoyl]-5-methoxyacetamido-2,4,6-triiodo benzoylaminoacetamido}-2,4,6-triiodoisochthalamide (lCJ-2089)

13.4 g (47.8 mmol) of sodium carbonate decahydrate are added to a solution of 11.9 g (15.6 mmol) of N,N'-bis-(2,3-dihydroxypropyl) -5-aminoacetamido -2,4,6- triiodoisophthalamide in 60 ml of DMA. It is stirred vigorously for about 10 minutes and 14.8 g (18.7 mmol) of the compound described in example 5a) are then added. stirring is continued during a whole night. The solid is filtered off and washed with methanol and the solvent is removed at reduced pressure. The residue is dissolved in 50 ml of methanol and the product precipitated with 500 ml of isopropanol. After filtering, the solid is washed with ethyl ether and dried in vacuo over phosphorus pentoxide, to obtain 18.8g of a slightly coloured solid (81%). This solid is hydrolysed in a hydrochloric acid medium stirring at room temperature overnight. It is neutralised and the. solvent is eliminated at reduced pressure. The residue is purified by preparative liquid chromatography. The fraction that contains the product is evaporated to dryness to obtain 10 g of product of with a chromatographic strength greater than 99% with an m.p. >260°C (which sinters at 254°C).

### Example 7

### N,N'-bis-(1-hydroxymethyl-2,3-dihydroxypropyl)-5-{3-[N-(1-hydroxymethyl-2,3-dihydroxypropyl)carbamoyl]-5-methoxyacetamido-2,4,6-triiodobenzoylaminoacetamido}-2,4,6-triiodo isophthalamide. (lCJ-1889)

### a) N,N'-bis-(1-hydroxymethyl-2,3-dihydroxypropyl)-5-aminoacetamido -2,4,6-triiodoisophthalamide.

50 g (0.064 moles) of 5-phthaloylaminoacetamido-2,4,6-triiodoisophthaloyl chloride are dissolved in 200 ml of DMF. 40g (0.14 moles) of Na₂CO₃.10H₂O are added and cooled to between 0 and 5°C. 22.6 g (0.14 moles) of 5-amino-2,2-dimethyl-5-hydroxy-1,2-dioxepane dissolved in 50 ml of DMF are then added and stirred at temperature room during 20 hours. Then, 12.44 ml (12.81 g, 0.28 moles) of hydrazine monohydrate are added and heated to 50-60°C for 8 hours. The suspended solid is filtered off and the solution is evaporated to dryness. The residue is dissolved in 2N HCl and heated to 50°C for 10 minutes. The suspended solid is filtered off and the solution evaporated to dryness. The residue is precipitated from methanol/isopropanol. 41.2 g (78%) with a m.p. of 170-185°C is obtained.

### b) N,N'-bis-(1-hydroxymethyl-2,3-dihydroxypropyl)-5-{3-[N-(1-hydroxymethyl-2,3-dihydroxypropyl)carbamoyl]-5-methoxi acetamido-2,4,6-triiodobenzoylaminoacetamido}-2,4,6-triiodo isophthalamide.

30.0 g (3.7g x 10-2moles) of the compound from example 5a) and 26 g (3.16 x 10-2moles) of the compound from example 7a) are dissolved in 168 ml of DMA. 27.1 g (9.48 x 10⁻² moles) of Na₂CO₃.10H₂O are added and stirred at temperature room for 20 hours. It is filtered and the solution evaporated to dryness. The residue is dissolved in methanol and precipitated with isopropanol. The crude product obtained is dissolved in methanol/water (1:1) and hydrolysed with 5% NaOH at 45-50°C. It is neutralised and evaporated to dryness. The crude product obtained is precipitated from methanol/isopropanol. 39g (yield:70%) was obtained with m.p. of 205-260°C.

### Example 8

### N,N'-bis-(1-hydroxymethyl-2,3-dihydroxypropyl)-5-{3-[N-(2,3-dihydroxypropyl)carbamoyl]-5-hydroxyacetamido-2,4,6 -triiodobenzoyl-N-methylaninoacetyl-N-nethylamino}-2,4,6-tri iodoisophthalamide. (lCJ-390)

### a) N,N'-bis-(7-hydroxy-4,4-dimethyl-3,5-dioxepanyl)-5-methyl-aminoacetyl-N-methylamino-2,4,6-triiodoisochthalamide.

180 g of the compound from example 3b) (192.37 mmol) are suspended in 1.450 ml of 30% methyl amine in ethanol are warmed to 40°C for 30 hours, during which the transformation is completed.
Methylamine is removed and it is then evaporated to dryness. Obtained: 171 g (molar yield: 95%).

### b) N,N'-bis-(1-hydroxymethyl-2 3-dihydroxypropyl)-5-methylaminoacetyl-N-methylamino-2,4,6-triiodoisophthalamide.

150 g (161.24 mmol) of the compound from example 8a) are dissolved in 500 ml de methanol. 35% hydrochloric acid is added until a pH=1 is reached. It is heated at 40°C until total hydrolysis has taken place. It is then neutralised with 50% sodium hydroxide and the sodium chloride that is formed is filtered off. It is then evaporated to dryness. Product obtained: 136.5 g (quantitative).

Thin-layer cromatography under the same conditions as in example 3a) retains the product at the origen.

### c) N,N'-bis-(1-hydroxymethyl-2,3-dihydroxypropyl)-5-{3-[N-(2,3-dihydroxypropyl)carbamoyl]-5-hydroxyacetamido-2,4,6-triiodobenzoyl-N-methylaminoacetyl-N-methylamino}-2,4,6-triiodoisophthalamide.

18.7 g (22.0 mmol) of the compound from example 8b) are suspended in 115ml of dimethylformamide, and 12.6 g of sodium carbonate decahydrate is added followed by 19.8 g (23.72 mmol) of the compound from example 2a). It is stirred at room temperature until the reaction is complete. (24 hours).

The salts are filtered off and it is evaporated to dryness. Then, the product is dissolved in 100 ml of methanol and the salts again filtered off. It is precipitated with 600 ml of isopropanol. Crude product obtained: 33.7 g (Yield: 90%).

The acetate groups are hydrolysed and the product purified by perparative liquid chromatography.
Pure product obtained: 15 g (molar yield 45%).

### Example 9

### N,N'-bis-(1-hydroxymethyl-2,3-dihydroxypropyl)-5-{3-[N-(2,3-dihydroxypropyl)carbamoyl]-5-methoxyacetamido -2,4,6-triiodobenzoyl-N-methylaminoacetyl-N-methylamino}-2,4,6-tri -iodoisophthalamide. (lCJ-490)

18.1 g (21.2g mmol) of the compound from example 8b) are dissolved in 80 ml of DMF, and 13.2 g (46.13 mmol) of sodium carbonate decahydrate are added. It is stirred for 5 min. and 18,5 g (22.94 mmol) of the compound from example la) are added. It is stirred at room temperature until the reaction is complete. (24 hours).
The inorganic salts are filtered off and it is evaporated to dryness. Then, it is then dissolved in 100 ml of methanol, filtered and precipitated with 600 ml of isopropanol. The product is then filtered off and dried.
Crude product obtained: 32 g (Yield 93%).
The acetate groups are hydrolysed and the product purified by preparative liquid chromatography.
Pure product obtained: 15 g (molar yield 46%)

### Example 10

### N,N'-bis-(1-hydroxymethyl-2,3-dihydroxypropyl)-5-{3-[N-(1-hydroxymethyl-2,3-dihydroxypropyl)carbamoyl]-5-methoxy acetamido -2,4,6-triiodobenzoyl-N-methylaminoacetyl-N-methylamino}-2,4,6-triiodoisophthalamide. (lCJ-690)

21.9 g (25.76 mmol) of the compound described in example 8b) are suspended in 100 ml of DMF, and 15.9 g (55.57 mmol) of sodium carbonate decahydrate are added. It is stirred for 5 minutes and 22 g (27.76 mmol) of the compound described in example 5a are added. Then, it is stirred at room temperature until the reaction is complete (30 hours). The inorganic salts are filtered off and it is evaporated to dryness. The product is dissolved in 100 ml of methanol (salts are filtered off) and it is then precipitated with 600 ml of isopropanol. It is filtered off and dried. Crude product obtained: 37 g (Yield: 90%)
The acetomide compound is hydrolysed and purified by preparative liquid chromatography.
Pure product obtained: 18 g (molar yield: 45%)

### Example 11

### N,N'-bis-(2,3-dihydroxypropyl)-5-{3-[N-(2,3-dihydroxypropyl)carbamoyl]-5-hydroxyacetamido-2,4,6-triiodo benzoyl-N-methylaminoacetyl-N-methylamino}-2,4,6-triiodo isophthalamide. (lCJ-1090)

### a) N,N'-bis-(2,3-diphidroxypropyl)-chloroacetyl-N-methylamino-2,4,6-triiodoisoehthalamide.

120 g (0.17 moles) of the compound from example 3a) are suspended in 360 ml DMF. 99.3 g (0.34 moles) of Na₂CO₃.10H₂O are added and the mixture cooled to between 0 and 5°C. A solution of 30.7 g (0.34 mol) of 3-amino-1,2-propanodiol in 90 ml of DMF is added and stirring is carried out for 20 hours . The solution is filtered and evaporated to dryness. The residue is dissolved in methanol and precipitated with ethyl ether.
12,5 g are obtained. (Yield: 94.3%). m.p.: 177-208°C.

### b) N,N'-bis-(2,3-dihydroxypropyl) 5-methylaminoacetyl-N-methylamino-2,4,6-triiodoisophthalamide

50 g (0,062 moles) of the compound in example 11a) are dissolved in 350 ml of methylamine/ethanol at 33% and the solution stirred at room temperature for 3 days. The solution is evaporated to dryness and the residue dissolved in methanol and precipitated with isopropyl alcohol.
Amount obtained: 33.3 g (Yield : 68%) m.p.: 178-205°C.

### c) N,N'-bis-(2,3-dihydroxypropyl)-5-{3-[N-(2,3-dihydroxypropyl)carbamoyl]-5-hydroxyacetamido-2,4,6-triiodobenzoyl -N-methylaminoacetyl-N-methylamino)-2,4,6-triiodoiso phthalamide.

30 g (0.038 moles) of the compound from example llb) and 34.87 g (0.041 moles) of the compound from example 2a) are dissolved in 190 ml of DMA. 32.61 g (0.11 moles) of Na₂CO₃,10H₂O are added and the mixture is stirred at room temperature for 20 hours. It is filtered, and the solution is evaporated to dryness . The residue is dissolved in methanol and precipitated with isopropanol. The crude product obtained is dissolved in methanol/water (1:1) and hydrolysed with 5% NaOH at 45-50°C. Then it is neutralised and evaporated to dryness. The crude product obtained (≈ 35 g) is purified by preparative liquid chromatography. Amount obtained: 20 g (Yield : 36.0%) m.p. : 256-268°C.

### Example 12

### N,N'-bis-(2,3-dihydroxypropyl)-5-{3-[N-(2,3-dihydroxypropyl)carbamoyl]-5-methoxyacetamido-2,4,6-triiodobenzoyl -N-methylaminoacetyl-N-methylamino}-2,4,6-triiodoisophthalamide (lCJ 1190)

22 g (0.027 moles) of the compound from example 11b) and 24.71 g (0.030 moles) of the compound from example la) are dissolved in 140 ml of DMA. 23.74 g (0.083 moles) of Na₂CO₃.10H₂O are added and stirred at room temperature for 20 hours. After filtering, the solution is evaporated to dryness. The residue is precipitated from methanol/ isopropanol. The product obtained (≈ 31.4 g) is dissolved in methanol/water (1:1) and hydrolysed with 5% NaOH at 45-50°C. It is neutralised and evaporated to dryness. The crude product obtained (27.6 g) is purified by preparative liquid chromatography. Amount obtained: 14 g (Yield : 35.1 % ). m.p.: 257-267°C.

### Example 13

### N,N'-bis-(2,3-dihydroxypropyl)-5-{3-[N-(2,3-dihydroxypropyl) carbamoyl]-5-hydroxyacetamido-2,4,6-triiodobenzoyl-N-(2,3-dihydroxypropyl)aminoacetamido}-2,4,6-triiodoisophthalamide. (lCJ - 191)

### a) 5-chloroacetylamino-2,4,6-triiodoisophthaloyl chloride

To a solution of 100 g (0.168 mol) 5-amino-2,4,6-triiodoisophthaloyl chloride in 300ml of DMA and cooled to 0-5°C, are slowly added 39.9 ml (0.502 mol) of chloroacetyl chloride. It is allowed to return to room temperature and is stirred for 6 hours. The reaction product is diluted with 700 ml of ethyl acetate and it is poured over 1000ml of water the organic phase is separated and extracted with 2x150 ml of ethyl acetate. The combined organic phases are washed with 2x150 ml of saturated sodium bicarbonate solution and with 250 ml of 10% sodium chloride solution. It is dried over anhydrous sodium sulphate, filtered and concentrated in vacuo until an abundant precipitate appears. After filtering it is washed with ethyl ether and dried in vacuo. 95 g (84%) of the desired product are obtained.
Thin-film chromatography (toluene) : Rf = 0.20

m.p.: 250-260°C (ethyl acetate)

### b) N,N'-bis-(2,3-dihydroxypropyl)-5-[N-(2,3-dihydroxypropyl) aminoacetamido]-2,4,6-triiodoisophthalamide.

A mixture of 80 g (0.120 mol) of the product described in example 13a), 119.2 g (0.416 mol) of sodium carbonate decahydrate and 76 g (0.834 mol)of 3-amino-1,2-propanodiol in 320 ml of DMF are heated to 50-60°C for 8 hours. It is filtered and concentrated in vacuo to dryness. The residue is dissolved in 240 ml of methanol, the salts that are precipitated are filtered off, and the product is precipitated in 2000 ml of isopropanol. It is filtered off and washed with ethyl ether.

The solid obtained is redissolved in 400 ml of methanol and precipitated in 2000 ml of ethanol. After filtering, it is washed with ethyl ether and dried in vacuo. In this way, 80 g (80%) of the desired product are obtained.
m.p. = 140-153°C

### c) N,N'-bis-(2,3-dihydroxypropyl)-5-{3-[N-(2,3-dihydroxypropyl)carbamoyl]-5-hydroxyacetamido-2,4,6-triiodobenzoyl --N-(2,3-dihydroxypropyl)aminoacetamido}-2,4,6-triiodo isophthalamide.

Following the general procedure described example 1b) and starting from the compound described in example 13b) (30 g, 0.036 mol) and that described in example 2a) (29.9 g, 0.036 mol).
Yield crude product: 75%.

### Example 14

### N,N'-bis-(2-hydroxyethyl)-5-{3-[N-(2,3-dihydroxypropyl)-carbamoyl]-5-hydroxyacetamido-2,4,6-triiodobenzoyl-N-(2, hydroxyethyl)aminoacetamido}-2,4,6-triiodoisophthalamide. (1CJ-103)

Following the general procedure described in example 1b) and starting from N,N'-bis(2-hydroxyethyl)-5-[N-(2-hydroxyethyl)aminoacetamido]-2,4-6-triiodoisophthalamide (described in DOS 2928417 ) (30 g, 0.040 mol) and the acid chloride described in example 2 a).
Yield of crude product:77%.

### Example 15

### N,N'-bis-(2,3-dihydroxypropyl)-5-{3-[N-(2,3-dihydroxypropyl)carbamoyl]-5-methoxyacetamido-2,4,6-triiodobenzoyl -N-(2,3-dihydroxypropyl)aminoacetamido}-2,4,6-triiodo isophthalamide. (lCJ-391)

Following the general procedure described in example 1b) and starting from the compound described in example 13b) (30g, 0.036 mol) and the acid chloride described in example la) (28.9 g, 0.036 mol).

### Example 16

### N,N'-bis-(2-hydroxyethyl)-5-{3-[N-(2,3-dihydroxypropyl)-carbamoyl]-5-hydroxyacetyl-N-(2-hydroxyethyl)amino-2,4,6-tri -iodobenzoyl-N-(2-hydroxyethyl)amino-acetyl-N-(2-hydroxyethyl) amino}-2,4,6-triiodoisophthalamide. (lCJ-291)

A mixture of the compound described in example 14 (ICJ-103) (23 g, 0.016 mol), trisodium phosphate dodecahydrate (32.4 g, 0.085 mol) and 2-chloroethanol (4.6 ml, 5.48 g, 0.068 mol) in methanol (60 ml), are refluxed for 2 to 3 days.
The crude product of the reaction is filtered, evaporated to dryness at reduced pressure, and the residue is purified by preparative liquid chromatography.
Yield: 55% m.p.: 215-238°C (decomposes)

### Example 17

### N,N'-bis-(2-hydroxyethyl)-5-{3-[N-(2,3-dihydroxypropyl)carbamoyl]-5-methoxyacetamido-2,4,6-triodobenzoyl-N-(2 -hydroxyethyl)aminoacetamido}-2,4,6-triiodoisophthalamide. (lCJ-105)

Following the general procedure described in example 1b) and starting from N,N'-bis(2-hydroxyethyl)-5-[N-(2-hydroxyethyl)-aminoacetamido]-2,4,6-triiodoisophthalamide described in DOS 2928417 (30 g, 0.040 mol) and the acid chloride described in example la) (32.4 g, 0.040 mol).
Yield of crude product: 80%.

### Example 18

### N,N'-bis-(2,3-dihydroxypropyl)-5-{3-[N-(2,3-dihydroxypropyl) carbamoyl]-5-hydroxyacetamido]-2,4,6-triiodobenzoylaminoacetamido}-2,4,6-triiodoisopchthalamide. (lCJ-790)

Following the general procedure described in example 1 b) and starting from N,N'-bis(2,3-dihydroxy-propyl)-5-amino-acetamido-2,4,6-triiodoisophthalamide (20g, 0.026 mol) and the acid chloride described in example 2a) (26.3 g, 0.031 mol). In this case, the crude is purified by recrystallisation in water.
Yield: 76% m.p.: 230-270 (decomposes)

### Example 19

### N,N'-bis-(1-hydroxymethyl-2,3-dihydroxypropyl)-5-{3-[N-(2,3-dihydroxypropyl)carbamoyl]-5-hydroxyacetamido-2,4,6 -triiodobenzoylaminoacetamido}-2,4,6-triiodoisophthalamide. (lCJ-1589)

28.0 g (3.3x10⁻²moles ) of N-(2,3-diacetoxypropyl)-5-acetoxyacetamido-2,4,6-triiodoisophthalanoyl chloride, described in example 2a) and 23.0 g (28x10⁻² moles) of the compound described in example 7a) are dissolved in 150 ml of DMA. 20.8 g (7.26x10⁻² moles) of Na₂CO₃.10H₂O are added and stirred at room temperature for 20 hours. The salts are filtered off and the solution evaporated to dryness. The residue is dissolved in methanol and precipitated in isopropanol. The product obtained (34.0 g) is dissolved in water/ methanol (1:1) and hydrolysed with 5% NaOH. It is neutralised and evaporated to dryness. The crude product obtained is precipitated in methanol/isopropanol. 27.5 g were obtained (Yield: 65.7%) m.p.: 200-255°C.

### Example 20

### N,N'-bis-(1-hydroxymethyl-2,3-dihydroxypropyl)-5-{3-[N-(2,3-dihydroxypropyl)carbamoyl]-5-methoxyacetamido-2,4,6 -triiodobenzoylaminoacetamido}-2,4,6-triiodoisolphthalamide. (lCJ-1689)

23.5 g (0,029 moles) N-(2,3-diacetoxypropyl)-2,4,6-triiodoisophthalamoyl chloride, described in example la) and 20.0 g (0.043moles) of the compound described in example 7a) are dissolved in 110 ml of DMA . 20.8 g (0.072 moles) of Na₂CO₃.10H₂O are added and stirred at room temperature for 20 hours. The salts in suspension are filtered off and the solution is evaporated to dryness. The residue is dissolved in methanol and precipitated in isopropanol. The product obtained (40 g) is dissolved in methanol/water (1:1) and hydrolysed with 5% NaOH at 45-50°C. The product is neutralised and evaporated to dryness. The crude product obtained is precipitated in methanol/isopropanol. 28.7 g of product is obtained (78.4%), m.p.: 200-280°C.

### Example 21

### N,N'-bis-(1-hydroxymethyl-2,3-dihydroxypropyl)-5-{3-[N-(2,3-dihydroxypropyl)carbomoyl]-5-methoxyacetamido-2,4,6-triiodo -benzoyl-N-methylaminoacetamido}-2,4,6-triiodoisophthalamide. (ICJ-1791)

### a) N,N'-bis-(1-hydroxymethyl-2,3-dihydroxypropyl)-5-methyl-aminoacetamido-2,4,6-triiodoisophthalamide.

Following the procedure used in example 24b) and starting from from 40 g (40.3 mmol) of N,N'-bis-(7-hidroxy-4,4-dimethyl-3,5-dioxepanyl)-5-chloroacetamido-2,4,6-triiodo-isophthalamide, (example 22a), and by means of the corresponding acid hydrolysis at 40°C, a syrupy residue is obtained which is precipitated by means of methanol-isopropanol. After filtering and drying with P₂O₅, 23.3 g (78%) of a slightly coloured solid is obtained.

### b) N,N'-bis-(1-hydroxymethyl-2,3-dihydroxypropyl)-5-5-{3-[N-(2,3-dihydroxypropyl)carbomoyl]-5-methoxyacetamido-2,4,6-triiodobenzoyl-N-methylaminoacetamido}-2,4,6-tri-iodoisophthalamide.

A suspension of 25 g (29.9 mmol) of N,N'-bis-(1-hydroxymethyl-2,3-dihydroxypropyl)-5-methylaninoacetanido-2,4,6-triiodoisophthalamide, (previous example) is stirred at room temperature for 10 hours with 24.1 g (29.9 mmcl) N-(2,3-diacetoxypropyl)-5-methoxyacetamido-2,4,6-triiodoiso-phthalamoyl chloride, (example la) and 17,1 g (59.8 mmol) of sodium carbonate decahydrate in 200 ml of DMA. It is filtered and the solvent removed under reduced pressure. The residue is dissolved in methanol and precipitated from isopropanol. The product is filtered and dried in vacuo, to obtain 62 g of a slightly coloured product. This solid is hydrolysed in an aqueous alcoholic solution with 20% NaOH. It is neutralised with 20% HC1 and the solvent is removed under reduced pressure. The residue is dissolved in methanol and precipitated from isopropanol. It is filtered and dried in vacuo to obtain 34 g (75%) of a slightly coloured solid which is purified by preparative liquid chromatography.

### Example 22

### N,N'-bis-(1-hydroxymethyl-2,3-dihydroxypropyl)-5-{3-[N-(2,3-dihydroxypropyl)carbamoyl]-5-methoxyacetamido-2,4,6-triiodo benzoyl-N-(2-hydroxyethyl)aminoacetamido}-2,4,6-triiodo isophthalamide. (ICJ-1691)

### a) N,N'-bis-(4,4-dimethyl-7-hydroxy-3,5-dioxepanyl)-5-chloro-acetamido -2,4,6-triiodoisophthalamide.

A suspension of 5-chloroacetamido-2,4,6-triiodoisophthaloyl chloride (134 g, 0.2 mol) described in example 13a), sodium carbonate decahydrate (172 g, 0.6 mol) and 6-amino-2,2-dimethyl-1,3-dioxepane-5-ol (32g, 0.2 mol) are heated to 55°C for 12-24 hours in acetone (400 ml). The solvent is removed in vacuo and the residue crushed with water. It is filtered off and dried in vacuo. Yield: 52%.

### b) N,N'-bis(1-hydroxymethyl-2,3-dihydroxypropyl)-5-[N-(2-hydroxyethyl)aminoacetamido]-2,4,6-triiodoisophthalamide.

A suspension of the compound (50g, 0.54 mol) as obtained in the previous section a), and 2-aminoethanol (8.1 ml, 7.98 g, 0.131 mol) is heated in ethanol (200 ml) at 50°C for 3 days.

When the transformation into N,N'-bis-(4,4-dimethyl-7-hydroxy-3,5-dioxepanyl)-5-[N-(2-hydroxyethyl)aninoacetamido]-2,4,6-triiodoisophthalamide - which is not isolated - is complete, the reaction medium is diluted with ethanol (200 ml) and hydrolysed by acidulating with hydrochloric acid. It is evaporated to dryness at reduced pressure and the residue is dissolved in methanol and precipitated in isopropanol. Filter and dry in vacuo.
Yield: 73%.

### c) N,N'-bis-(1-hydroxymethyl-2,3-dihydroxypropyl)-5-{3-[N-(2,3-dihydroxypropyl)carbamoyl]-5-methoxyacetamido-2,4,6 -triiodobenzoyl-N-(2-hydroxyethyl)aninoacetamido}-2,4,6 -triiodo-isophthalamide.

Following the general procedure described in example 1b) and starting from the compound described in the previous section b) (23 g, 0.027 mol) and with the acid chloride in example la) (21.41 g, 0.027 mol).
Yield of crude product: 70%.

### EXAMPLE 23

### N,N -bis-(2,3-dihydroxypropyl)-5-{3-[N-(2,3-dihydroxypropyl) carbamoyl]-5-acetamido-2,4,6-triiodobenzoyl-N-(2,3-dihydroxypropyl)aminoacetyl-N-methylamino}-2,4,6-triiodoisophthalamide. (lCJ - 1891)

### a) N,N'-bis-(2,3-dihydroxypropyl)-5-{3-[N-(2,3-dihydroxypropyl) aminoacetyl-N-methylamino]}-2,4,6-triiodoisophthalamide.

This is prepared following the procedure described in Example 13b), and starting from 5-chloroacetyl-N-methylamino-2,4,6-triiodo-isophthaloyl chloride described in Example 3 (82.3 g, 0.120 mol), sodium carbonate decahydrate (120.1 g, 0.420 mol) and 3-amino-1,2-propanodiol (54.6 g, 0.600 mol).
Yield: 78%.

### b) N,N'-bis-(2,3-dihydroxypropyl)-5-{3-[N-(2,3-dihydroxypropyl)carbamoyl]-5-acetamido-2,4,6-triiodobenzoyl-N-(2,3-dihydroxypropyl)aminoacetyl-N-methylamino}-2,4,6-triiodoisophthalamide.

Following the general procedure described in Example 1b) and starting from the compound described in the previous section a) (40 g, 0.047 mol) and the chloride of the acid described in Example 36b) (36.53 g, 0.047 mol).
Yield: 76%.

### EXAMPLE 24

### N,N'-bis-(2,3-dihydroxypropyl)-N,N'-dimethyl-5-{3-[N-(2,3-dihydroxypropyl)carbamoyl]-5-hydroxyacetamido-2,4,6-triiodo benzoyl-N-methylaminoacetyl-N-methylamino}-triiodo isophthalamide. (lCJ - 890)

### a) N,N'-bis-(2,3-dihydroxypropyl)-N,N'-dimethyl-5-chloroacetyl-methylamino-2,4,6-triiodoisophthalamide.

118 g (0.77 moles) 5-chloroacetyl-N-methylamino-2,4,6-triiodoisophthaloyl chloride are suspended in 240 ml of DMF. 97.28 g (0.34 moles) of Na₂CO₃.10H₂O are added and the solution cooled to between 0 and 5°C.

Once this temperature has been reached, 39.7 g (0.34 moles) of N-methyl-3-aminopropano-diol is added dropwise, dissolved in 65 ml of DMF. Once the addition has been ended, it is allowed to return to room temperature and it is stirred for 20 hours. The insoluble salts are filtered off and the solution is evaporated to dryness. The resulting residue is dissolved in 200 ml of methanol and precipitated by addition into one litre of ethyl ether. The solid that is precipitated is filtered off, washed with ether and dried in vacuo. 131.2 g are obtained.
(Yield: 93.7%). m.p.: 130-165°C.

### b) N,N'-bis-(2,3-dihydroxypropyl)-N,N'-dimethyl-5-methylaminoacetyl-N-methylamino-2,4,6-triiodoisophthalamide

A solution of 70g (25 mmol) of N,N'-bis-(2,3-dihydroxypropyl)-N,N'-dimethyl-5-chloroacetyl-N-methylamino-2,4,6-triiodo-isophthal-amide and 70 ml of 33% alcoholic methylamine in 140 ml of ethanol is heated at 40°C fo 30 hours. It is cooled to room temperature and filtered. The solvent is removed under reduced pressure. The residue is dissolved in 100 ml of methanol and this is added to 200 ml isopropyl alcohol. Once the addition has been finished, it is stirred for a few minutes and filtered. The solid is washed with isopropyl alcohol and ethyl ether, and then dried in vacuo over P₂O₅. 41.9 g (52%) of a colourles solid is obtained.

### c) N,N'-bis-(2,3-dihydroxylpropyl)-N,N'-dimethyl-5-{3-[N-(2,3-dihydroxypropyl)carbamoyl]-5-hydroxyacetamido-2,4,6-triiodobenzoyl-N-methylamino acetyl-N-methylamino}triiodoisophthalamide

To a solution of 2.3g (26 mmol) of N,N'-bis-(2,3-dihydroxypropyl)-N,N'-dimethyl-5-methylaminoacetyl-N-methylamino-2,4,6-triiodoisophthalamide in 270 ml of DMA are added 22.3 g (78 mmol) of Na₂CO₃.10H₂O. There is then added 22.7 g, (28.6 mmol) of the chloride described in Example 2a), the mixture is stirred at room temperature until the following day and it is evaporated to dryness at reduced pressure (weight of the residue: 50.2 g). The residue is dissolved in 1:1 aqueous methanol and is hydrolysed with 5% NaOH at 40°C. It is cooled to room temperature and neutralised with 20% HCl. The solvent is removed at reduced pressure and 100 ml of methanol is added to the residue. The methanol solution is added to 1000 ml of isopropyl alcohol. Once the addition has been ended, it is stirred for a few minutes and filtered. The product is dried in vacuo over P₂O₅, and purified by preparative liquid chromatography.

### EXAMPLE 25

### N,N'-bis-(2,3-dihydroxypropyl)-N,N'-dimethyl-5-{3-[N-(2,3-dihydroxypropyl)carbamoyl]-5-methoxyacetamido-2,4,6-triiodo benzoyl-N-methylaminoacetyl-N-methylamino}-2,4,6-triiodo isophthalamide (lCJ - 990)

To a solution of 21.3 g (26 mmol) of the compound from example 24b) in 270 ml of DMA, is added 22.3 g (78 mmol) of Na₂CO₃.10H₂O and 23.06 g (28.6 mmol) of the compound from Example la). It is stirred at room temperature overnight. It is filtered and evaporated to dryness at reduced pressure. (weight of the residue 51.2 g).

The residue is dissolved in 50% aqueous methanol and hydrolysed at 40°C with 20% NaOH. It is cooled to room temperature and neutralised with 20% HCl. The solvent is removed at reduced pressure and the residue treated with 100 ml of methanol. The methanolic solution is precipitated with 1000 ml of isopropyl alcohol. After stirring for 5 minutes it is filtered, then dried in vacuo and filtered off, followed by drying in vacuo over P₂O₅. 30 g of a slightly coloured solid are obtained which is purified by preparative liquid chromatography. 19.9 g (51%) of a colourles solid is obtained.

### EXAMPLE 26

### N,N'-bis-(2,3-dihydroxypropyl)-N,N'-dimethyl-5-{3-[N-(2,3-dihydroxypropyl)carbamoyl]-5-hydroxyacetamido-2,4,6-triiodo benzoylaminoacetyl-N-methylamino}-2,4,6-triiodoiso phthalamide (lCJ - 1290)

20 g (0.023 moles) of the compound from Example 2a) and 17.51 g (0.021 moles) of N,N'-bis-(2,3-dihydroxypropyl)-N,N'-dimethyl-5-aminoacetyl-methylamino-2,4,6-triiodoisophthalamide are dissolved in 112 ml of DMA. 18.12g. (0.065 moles) Na₂CO₃.10H₂O are added and it is left to stir at room temperature for 20 hours. The insoluble salts are filtered off and the solution is evaporated to dryness. The residue is dissolved in methanol and precipitated in isopropanol. The product obtained (≈ 24 g)is dissolved in methanol/water (1:1) and hydrolysed with 5% NaOH at 45-50°C. It is neutralised and evaporated to dryness. The crude product obtained is purified by preparative liquid chromatography. 13.2 g. is obtained.
(Yield: 41.2 %) m.p.: 110 - 210°C.

### EXAMPLE 27

### N,N'-bis-(2,3-dihydroxypropyl)-N,N'-dimethyl-5-{3-[N-(2,3-dihydroxypropyl)carbamoyl]-5-methoxyacetamido-2,4,6 -triiodobenzoylaminoacetyl-N-methylamino}-2,4,6-triiodo isophthalamide (lCJ - 1390)

19.3 g (0.024 moles) of the compound from example 1a) and 17.5 g (0.021 moles) of the compound described in DOS 2 928 417 are dissolved in 112 ml of DMA, 18.3 g (0.065 moles) of Na₂CO₃.10H₂O are added and stirred at room temperature for 20 hours. The insoluble salts are filtered off and the solution evaporated to dryness. The product obtained (≈ 27 g) is dissolved in (1:1) water/methanol and hydrolysed with 5% NaOH at 45-50°C. It is neutralised and evaporated to dryness. The crude product is purified by preparative liquid chromatography. i4.5 g. are obtained. (Yield: 44.8%) m.p. Sinters and decomposes between 140 and 230°C.

### EXAMPLE 28

### N,N'-bis-(2,3-dihydroxypropyl)-5-{3-[N-(2,3-dihydroxypropyl) carbamoyl]-5-methoxyacetamido-2,4,6-triiodobenzoylaminoacetyl-N-methylamino}-2,4,6-triiodoisophthalamide. (lCJ - 1590)

### a) N,N'-bis-(2,3-dihydroxypropyl)-5-aninoacetyl-N-methylamino-2,4,6-triiodoisophthalamide

50 g (0.062 moles) of 5-phthaloylamino-acetyl-N-methylamino-2,4,6-triiodoisophthaloyl chloride are dissolved in 150 ml of DMF. The mixture is cooled to between 0 and 5°C and 36.48g of Na₂CO₃.10H₂O (0.124 moles) and 11.4 g (0.124 moles) of 3-amino-1,2-propanodiol dissolved in 50 ml of DMF, are added. It is left stirring at room temperature for 24 hours. 12 ml (0.25 moles) of hydrazine hydrate are added. The mixture is heated to 50°C for 3 hours. The suspended solid is filtered off and the solution evaporated to dryness. The residue is dissolved in 2N HCl and heated at 50°C for 10 minutes. The suspended solid is filtered off and the solution evaporated to dryness. The residue is precipitated from methanol/ isopropanol. 24.0 g are obtained.
(Yield: 50%). m.p.: 220-230°C.

### b) N,N'-bis-(2,3-dihydroxypropyl)-5-{3-[N-(2,3-dihydroxypropyl)carbamoyl]-5-methoxyacetamido-2,4,6-triiodobenzoylaminoacetyl-N-methylanino}-2,4,6-triiodoisophthalamide

21.72 g (0.027 moles) of the compound described in Example 1a) and 19.0 g (0.024 moles) of N,N'-bis-(2,3-dihydroxypropyl)-5-aminoacetyl-N-methylamino-2,4,6-triiodoisophthalamide are dissolved in 122 ml of DMA. 21.03 g (0.073 moles) of Na₂CO₃.10H₂O are added, and stirred at room temperature for 20 hours. The insoluble salts are filtered off and the solution evaporated to dryness. The residue is dissolved in methanol and precipitated in isopropyl [alcohol]. The product obtained (≈ 32 g ) is dissolved in (1:1) methanol/water and hydrolysed with 5% NaOH at 45-50°C. The solution is neutralised and evaporated to dryness. The residue is recrystallised from water. 14.4 g. of product is obtained. (Yield : 40.2 % ), m.p.: Sinters and decomposes between 130-210°C.

### EXAMPLE 29

### N,N,N',N'-tetrakis-(2-hydroxyethyl)-5-{3-[N-(2,3-dihydroxypropyl)carbamoyl]-5-hydroxyacetamido-2,4,6-triiodobenzoyl-N-methylaminoacetyl-N-methylamino}-2,4,6-triiodoisophthalamide (lCJ-1690)

### a) 5-chloroacetylmethylamino-N,N,N',N'-tetrakis-(2-hydroxyethyl)-2,4,6-triiodoisophthalamide

To a solution of 27.5 g (40 mmol) of the compound in Example 3a) in 85 ml of DMF is added 22.9 g (80 mmol) of Na₂CO₃.10H₂O. It is cooled to 10°C and to it is added a solution of 8.83 g (84 mmol) of diethanolamine dissolved in 8 ml of DMF. Once the addition is completed, it is warmed to ambient temperature for three hours and the solvent is removed under reduced pressure to obtain a solid residue (16 g). The residue is dissolved in MeOH and 500 ml of ethyl ether are added. Once the addition is complete, it is stirred for a moment and filtered, and dried in vacuo over P₂O₅. 28 g of a slightly coloured solid are obtained which is purified by c.c. flash (acetone/MeOH 2:1). 19.6 g (60%) of a colourless solid are obtained with a m.p. of 60-98°C.

### b) 5-methylaminoacetyl-N-methylamino-N,N,N',N'-tetrakis-(2--hydroxyethyl)-2,4,6-triiodoisophthalamide

A suspension of 70 g (85 mmol) of the compound in Example 29a) in 140 ml of ethanol and 70 ml of a 33% methylamine in ethanol solution are stirred together at 40°C for 30 hours. It is filtered and evaporated to dryness. The residue is dissolved in 120 ml of methanol and it is added to 900 ml of isopropyl alcohol. When the addition has ended , it is filtered and dried in vacuo over P₂O₅. 21.8 g of a colourless solid is obtained which is purified by flash c.c. (CHCl₃/MeOH 3:2). 15.1 g (22%) of a colourless solid is obtained.

### c) N,N,N',N'-tetrakis-(2-hydroxyethyl)-5-{3-[N-(2,3-dihidroxy-propyl)carbamoyl]-5-hydroxyacetamido-2,4,6-triiodobenzoyl-N-methylaminoacetyl-N-methylamino}-2,4,6-triiodo-isophthalamid

To a solution of 21.3 g (26 mmol) of the compound of Example 29b) in 270 ml of DMA is added 22.3 g. (78 mmol) Na₂CO₃.10H₂O and 22.7 g (28.6 mmol) of the compound of Example 2a).

It is stirred at room temperature overnight and filtered at reduced pressure (weight of residue: 52.3 g.).

The residue is dissolved in 50% aqueous methanol. It is then hydrolysed at 40°C with 20% NaOH, cooled to room temperature and neutralised with 20% HCl. The solvent is removed under reduced pressure and the residue is treated with 200 ml of methanol. The methanol solution is precipitated in 1000 ml of isopropyl alcohol. It is filtered off and dried in vacuo with P₂O₅ to obtain 34.6 of a yellowish solid which is purified by preparative liquid chromatography. 16.6g. (43%) of a colourless solid is obtained.

### EXAMPLE 30

### N,N,N',N', -tetrakis-(2-hydroxyethyl)-5-{3-[N-(2,3-dihydroxypropyl)carbamoyl]-5-methoxyacetamido-2,4,6-triiodobenzoyl-N -methylaminoacetyl-N-methylamino}-2,4,6-triiodo-isophthalamide (lCJ - 1790)

To a solution of 17 g (20.8 mmol) of the compound from Example 29b) in 215 ml of DMA, are added 17.85 g (62.4 mmol) Na₂CO₃.10H₂O and 18.5 g (22.9 mmol) of the compound from Example la), and the whole is stirred at room temperature for a night. It is filtered and evaporated to dryness under reduced pressure (weight of the residue 29 g.) .

The residue is dissolved in 50% aqueous methanol, and hydrolysed at 40°C with 20% NaOH. It is cooled to room temperature and neutralised with 20% HCl. The solvent is removed under reduced pressure. The residue is treated with methanol and the methanol solution is precipitated with isopropyl alcohol. Once the precipitation has finished, it is stirred for a moment, the product is filtered off and dried in vacuo over P₂O₅. 23.7 g of a slightly coloured solid is obtained which is purified by preparative liquid chromatography.
15.6 g (50%) of a colourless solid is obtained.

### EXAMPLE 31

### N,N,N',N'-tetrakis(2-hydroxyethyl)-5-{3-[N(2,3-dihydroxypropyl)carbamoyl]-5-methoxyacetamido-2,4,6-triiodobenzoyl aminoacetamido}-2,4,6-triiodoisophthalamide (lCJ - 1890)

### a) 5-phthalimidoylacetamido-N,N,N',N'-tetra-kis-(2-hydroxyethyl)-2,4,6-triiodoisophthalamide

To a solution of 31.3 g (40 mmol) of 5-phthalimidoylacetamido-2,4,6-triiodoisophthaloyl chloride in 85 ml of DMF is added 22.9 g (80 mmol) of Na₂CO₃.10H₂O. It is cooled to 10°C and 8.83 g (84 mmol) of diethanolamine in 8 ml of DMF are added. Once the addition has been completed, it is warmed to room temperature and stirred overnight. It is filtered and evaporated to dryness at a reduced pressure. Finally, it is broken up in 50 ml of water. It is filtered off and dried in vacuo over P₂O₅, to obtain 20.2 (55%) of a colourless solid.

### b) 5-aminoacetamido-N,N,N',N'-tetrakis-(2-hydroxyethyl)-2,4,6-triiodoisophthalamide

A suspension of 60 g (65.2 mmol) of the compound from Example 31a) in 210 ml of ethanol is refluxed, and 13.05 g (260.8 mmol) of NH₂-NH₂.H20 are added, the product of the reaction redissolving after a few minutes. After 7 hours, it is cooled to room temperature and the solvent is removed under reduced pressure from the reaction medium. The residue is stirred at 40°C with 250 ml of 5% HCl for one hour. It is cooled to room temperature and filtered. The filtrate is evaporated to dryness under reduced pressure. 50 ml of MeOH are added and it is then left in the refrigerator overnight. The hydrazine hydrochloride is filtered off and the filtrate is concentrated to 2/3 of its initial volume, and it is again left in the refrigerator overnight. The product is filtered off and washed with methanol (1 x 10 ml) and ethyl ether (1 x 10 ml) and dried in vacuo over P₂O₅. 18.1 g (74%) of a colourless solid is obtained.

### c) N,N,N',N'-tetrakis-(2-hydroxyethyl)-5-{3-[N-(2,3-dihydroxypropyl)carbamoyl]-5-methoxyacetamido-2,4,6-triiodo-benzoylaminoacetamido}-2,4,6-triiodoisophthalamide.

To a solution of 20 g (25.3 mmol) of the compound from Examole 31c) in 200 ml of DMA are added 21.7 g (75.9 mmol) Na₂CO₃.10H₂O and 22.5 g (27.8 mmol) of the compound from Example 1a). It is stirred at room temperature overnight. It is filtered and the solvent removed under reduced pressure (weight of residue 52 g).

The residue is dissolved in 200 ml of 50% aqueous methanol and hydrolysed with 20% NaOH al 40°C. It is cooled to room temperature and neutralised with 20% HCl. The solvent is removed under reduced pressure. The residue is purified by recrystallisation (MeOH/H₂O) to obtain 24.1 g (65%) of a colourless solid.

### EXAMPLE 32

### N,N'-bis-(2-hydroxyethyl)-5-{3-[N-(2,3-dihydroxypropyl)carbamoyl]-5-methoxyacetyl-N-(2-hydroxyethyl)amino-2,4,6 -triiodobenzoyl-N-(2-hydroxyethyl)aminoacetyl-N-(2-hydroxyethyl) amino}-2,4,6-triiodoisophthalamide (lCJ - 491)

Following the general procedure described for the compound of Example 16) and starting from: 30 g (0.021 mol) of the compound from Example 17, 42.3 g (0.111 mol) trisodium phosphate dodecahydrate and 6.0 ml (7.2 g, 0.089 mol) of 2-chloroethanol. Yield: 45% m.p.: 220 -240°C

### EXAMPLE 33

### N,N'-bis-(2,3-dihydroxypropyl)-5-{3-[N-(2,3-dihydroxypropyl)carbamoyl]-5-methoxyacetyl-N-(2-hydroxyethyl)amino-2,4,6-triiodobenzoyl-N-(2-hydroxyethyl)aminoacetyl-N-(2-hydroxyethyl)amino)-2,4,6-triiodoisophthalamide (lCJ - 691)

### a) N,N'-bis-(2,3-diacetoxypropyl)-5-chloroacetamido-2,4,6-triiodoisophthalamide

To a solution of 87.3 g (0.1 mol) of 5-amino-N,N-bis-(2,3-diacetoxypropyl)-2,4,6-triiodo-isophthalamide in 260 ml of DMA, cooled to between 0-5°C, is slowly added 23.9 ml (33.9 g, 0.3 mol) acetoxyacetyl chloride. It is allowed to return to room temperature and is stirred for 5 - 6 hours.

9 ml of water are added to destroy the excess of acid chloride and it is poured into 1,250 ml of cold water. The water is decanted off and the grease that is formed is dissolved in 450 ml ethyl acetate. The water is extracted with 3 x 350 ml of AcOEt. The combined organic extracts are washed with : a) 2 x 250 ml 10% sodium bicarbonate solution and b) 250 ml of a 10% sodium chloride solution. It is dried over anhydrous magnesium sulphate, filtered and the solvent removed under reduced pressure. The solid obtained is dried in vacuo.
Yield: 95 - 99%
m.p.: 190-205°C.

### b) N,N'-bis-(2,3-dihydroxypropyl)-5-[N-(2-hydroxyethyl)amino]acetamido-2,4,6-triiodoisophthalamide

A solution of 9.50 g (10 mmol) of the compound from Example 33a) and 4.89 g (80 mmol) 2-aminoethanol in 20 ml of ethanol are heated overnight at 50°C. The mixture is cooled to room temperature, filtered and dried in vacuo, with P₂O₅, to obtain 7.7 g (95%) of a colourless solid with a m.p. = 216-220°C.

### c) N,N'-bis-(2,3-dihydroxypropyl)-5-{3-[N-(2,3-dihydroxypropyl) carbamoyl]-5-methoxyacetamido-2,4,6-triiodobenzoyl-N-(2-hydroxyethyl)aminoacetylamino}-2,4,6-triiodoisophthalamide

To a solution of 30 g (17.2 mmol) of the compound from Example 33b) in 180 ml of DMA are added 10.01 g (17.2 mmol) of the compound from Example la) and 21.3 g (74.4 mmol) of Na₂CO₃.10H₂O. The mixture is stirred vigorously at room temperature overnight. It is filtered and the solvent is evaporated under reduced pressure until dryness. The residue is dissolved in 150 ml of MeOH which is added to 900 ml of isopropyl alcohol. It is filtered off and dried in vacuo with P₂O₅. 50 g of a lightly coloured solid are obtained which is purified by breaking it up in 500 ml of isopropyl alcohol and refluxing for 1 hour. It is filtered and washed with ethyl ether (2 x 50 ml), and then dried in vacuo with P₂O₅ to obtain 41 g of a colourless solid.

These 41 g are dissolved in 200 ml of 50% aqueous methanol. It is hydrolysed at 40°C with 20% NaOH, cooled to room temperature and neutralised with 20% HCl. The solvent is removed under reduced pressure (weight of residue 39 g). 60 ml of methanol are added and this methanolic solution is added over 500 ml of isopropyl alcohol. After filtering and drying in vacuo with P₂O₅, 36 g (65%) of a colourless solid are obtained.

### d) N,N'bis(2,3-dihydroxypropyl)-5-{3-[N-(2,3-dihydroxypropyl) carbamoyl]-5-methoxyacetyl-N-(2-hydroxyethyl)amino-2,4,6-triiodobenzoyl-N-(2-hydroxyethyl)aminoacetyl-N-(2-hydroxyethyl)amino}-2,4,6-triiodoisophthalamide

To a solution of 26 g (17.4 mmol) of the compound from Example 33c) in 65 ml of methanol, are added 34.8 g (91.52 mmol) of Na₃PO₄.12H₂O. It is stirred for a short while and 7.35 g (91.29 mmol) of 2-chloroethanol are added and heated to 60°C. Stirring is continued at 60°C overnight. It is filtered and the alcoholic solvent is removed by evaporation under a reduced pressure. The residue (26.8 g) is purified by preparative liquid chromatography, to obtain 11 g (40%) of a colourless solid.

### EXAMPLE 34

### N,N'-bis-(2,3-dihydroxypropyl)-5-{3-[N-(2,3-dihydroxypropyl) carbamoyl]-5-hydroxyacetyl-N-(2-hydroxyethyl)amino-2,4,6-triiodobenzoylaminoacetyl-N-(2-hydroxyethyl)amino}-2,4,6 -triiodoisophthalamide (lCJ - 791)

To a solution of 16 g (11.16 mmol) of the compound from Example 18, in 40 ml of methanol are added 22.3 g (58.70 mmol) of Na₃PO₄.12H₂O. It is stirred for a short while and heated to 60°C, and 4.67 g (58.00 mmol) of 2-chloroethanol are added. Stirring is continued at 60°C overnight. It is then filtered and the alcoholic solvent is removed from the solution and washings by evaporation at reduced pressure. The residue is purified by preparative liquid chromatography to obtain 8 g (47%) of a colourless solid.

### EXAMPLE 35

### N,N'-bis-(2,3-dihydroxypropyl)-5-{3-[N-(2,3-dihydroxypropyl)carbamoyl]-5-methoxyacetyl-N-(2-hydroxyethyl)amino-2,4,6-tri iodobenzoyl-N-methylaminoacetyl-N-(2-hydroxyethyl)amino}-2,4,6 triiodoisophthalamide (lCJ - 1091)

### a) N,N'-bis-(2,3-dihydroxypropyl)-5-methylaminoacetamido-2,4,6-triiodoisophthalamide

9.50 g (10 mmol) of N,N'-bis-(2,3-dihydroxypropyl)-5-chloroacetamido-2,4,6-triiodo-isophthalamide, 20 ml of 33% ethanolic methylamine and 20 ml of ethanol, are heated at 40°C for 8 hours. The reaction product dissolves in a short while, and during the course of the process precipitates abundantly. It is filtered off and dried in vacuo with P₂O₅ to obtain 7 g (90%) of a colourless solid.

### b) N,N'-bis-(2,3-dihydroxypropyl)-5-{3-[N-(2,3-dihydroxypropyl)carbamoyl]-5-methoxyacetamido-2,4,6-triiodobenzoyl-N-methylaminoacetamido}-2,4,6-triiodoisophthalamide

To a solution of 28.9 g (17.2 mmol) of the compound from Example 35a) in 180 ml of DMA are added 10.01 g (17.2 mmol) of the compound from Example la) and 21.3 g (74.4 mmol) Na₂CO₃.10H₂O. It is stirred at 60 C overnight and the solvent is evaporated under reduced pressure until dryness . The residue is dissolved in 120 ml of MeOH and added to 650 ml of isopropyl alcohol. It is filtered and dried in vacuo with P₂O₅. 51.2 g of a slightly coloured solid are obtained. This solid is dissolved in 250 ml of 50% aqueous methanol and hydrolysed with 20% NaOH at 40°C. It is cooled to room temperature and neutralised with 20% HCl. The solvent is removed under reduced pressure (weight of residue 49 g), 150 ml of methanol are added and the methanolic solution is added to 650 ml of isopropyl alcohol. Once the addition has been ended, it is filtered. It is dried in vacuo over P₂O₅, to obtain 39 g (72%) of a colourless solid.

### c) N,N'-bis-(2,3-dihydroxipropyl)-5-{3-[N-(2,3-dihydroxypropyl)carbamoyl]-5-methoxyacetyl-N-(2-hydroxyethyl)amino-2,4,6-triiodobenzoyl- N-methylaminoacetyl-N-(2-hydroxy-ethyl)amino}-2,4,6-triiodoisophthalamide

To a solution of 31 g (21.2 mmol) of the compound from Example 35b) in 80 ml of methanol is added 42.4 g (111.51 mmol), of Na₃PO₄.12H₂O It is heated to reflux and 7.2 g (39.04 mmol) of 2-chloroethanol are added. Heating is continued with reflux overnight. It is filtered and the solvent removed under reduced pressure. The residue (16.6 g) is purified by preparative liquid chromatography, to obtain 18 g (55%) of a colourless solid.

### EXAMPLE 36

### N,N-bis-(2,3-dihydroxypropyl)-5-{3-[N-(2,3-dihydroxypropyl) carbamoyl]-5-acetamido-2,4,6-triiodobenzoyl-N-(2,3-dihidroxypropyl)aminoacetamido}-2,4,6-triiodoisophthalamide (lCJ-1391)

### a) N-(2,3-diacetoxypropyl)-5-acetamido-2,4,6-triiodoisophthalamic acid

A mixture of 316 g (0.50 ml) N-(2,3-dihydroxypropyl)-5-amino-2,4,6-triiodoisophthalamic acid, 3.2 ml concentrated sulphuric acid and 390 ml (421 g 4.12 mol) of acetic anhydride are refluxed in 1,200 ml of ethyl acetate. When the reaction has ended, the solid that is formed is filtered off, washed with ethyl ether and dried in vacuo to constant weight.
Yield: 93 %
M.p.: 263-264°C (d)

### b) N-(2,3-diacetoxypropyl)-5-acetamido-2,4,6-triiodoisophthalamoyl chloride

To a solution of 350 g (0.462 mol) of the compound described in Example 36a) in 1,250 ml of DMA cooled to 5-10°C are slowly added 134 ml (219.6 g 1.85 mol) of thionyl chloride. When the addition has ended, stirring is maintained at approximately 10°C for 1 hour, it is diluted with 3,000 ml of ethyl acetate and it is poured into 3,000 ml of water. The organic phase is separated and the aqueous phase is extracted with 2,000 ml of ethyl acetate. The combined organic phases are washed twice with 1,000 ml of a saturated solution of sodium bicarbonate and once with 1,000 ml of a 10% solution of sodium chloride. The extract is dried over anhydrous sodium sulphate, it is then filtered and treated for 2 hours with 30 g active carbon. It is filtered and evaporated to dryness under reduced presion and the resulting residue is broken up by stirring with 2,000 ml of petroleum ether for 3 hours. It is filtered and dried in vacuo to constant weight.
Yield: 90%
m.p.: 203-208°C (AcOEt) (d)

### c) N,N-bis-(2,3-dihydroxypropyl)-5-{3-[N-(2,3-dihydroxypropyl) carbamoyl]-5-acetamido-2,4,6-triiodobenzoyl-N-(2,3-dihydroxypropyl)aminoacetanido}-2,4,6-triiodoisophthalamide

It is obtained and purified following the same general procedure described for the compound from Example 1b), starting from: 30 g (0.036 mol) of the compound described in Example 13b ) y 27.8 g (0.036 mol) of the N-(2,3-diacetoxypropyl)-5-acetamido-2,4,6-triiodoisophthalamoyl chloride described in Example 36b).
Yield: 45%
m.p.: 255-268°C (d)

### EXAMPLE 37

### N,N'-bis-(2,3-dihydroxypropyl)-5-{3-[N-(2,3-dihydroxypropyl) carbamoyl]-5-acetamido-2,4,6-triiodobenzoyl-N-methylacetyl-N-methylamino}-2,4,6-triiodoisophthalamide (lCJ - 1491)

44.0 g (0.056 moles) of the compound from Example 36b ) and 47.7 g (0.056 moles) of the compound from Example described in 11b) are dissolved in 264 ml of DMA. 32.04 g (0.11 moles) of Na₂CO₃.10H₂O are added, and it is stirred at room temperature for 20 hours. The insoluble salts are filtered off and the solution is evaporated to dryness. The residue is dissolved in methanol and precipitated from isopropanol. The product obtained (≈ 65.5 g) is dissolved in (1:1) methanol/water and hydrolysed with 5% NaOH at 45-50°C. It is neutralised and evaporated to dryness. The crude product obtained is purified by preparative liquid chromatography. 20 g of product are obtained.
Yield: 32.7%
m.p.. Decomposes and sinters from 160°C.

### EXAMPLE 38

### N,N'-bis-(1-hydroxymethyl-2,3-dihydroxypropyl)-5-{3-[N-(2,3-dihydroxypropyl]carbamoyl]-5-acetamido-2,4,6-triiodobenzoyl -N-methylacetyl-N-methylamino}-2,4,6-triiodoisophthalamide (1CJ - 1591)

50 g (5.5 x 10⁻² moles) of the compound from Example 8a) and 42.83 g (5.5 x 10⁻² moles) of the compound from Example 36b) are dissolved in 300 ml of DMA. 31.6 g (0.11 moles) of Na₂CO₃.10H₂O are added and the mixture is stirred at room temperature for 20 hours. The insoluble salts are filtered off and the solution is evaporated to dryness. The residue is dissolved in methanol and precipitated from isopropanol. The product obtained (≈ 69.2 g) is dissolved in (1:1) methanol/water and is hydrolysed with 5% NaOH at 45 -50°C. It is neutralised and evaporated to dryness and the crude product obtained is purified by preparative liquid chromatography. 25.5 g of product are obtained.
Yield: 30.8 % . m.p.: Sinters and decomposes above 170°C.

### Example 39

### N,N'-bis-(2,3-dihydroxypropyl)-5-{3-[N-(2,3-dihydroxypropyl) carbamoyl]-5-methoxyacetamido-2,4,6-triiodobenzoyl--N-(2-hydroxy-3-methoxypropyl)aminoacetyl-N-methylamino} -2,4,6-triiodoisophthalamide. (lCJ-1991)

### a) N,N'-bis-(2,3-dihydroxypropyl)-5-[N-(2-hydroxy-3-methoxypropyl)aninoacetyl-N-methylamino]-2,4,6-triiodoisophthalamide

50 g (0.062 moles) of the compound from example 11a) and 34.3 g (0.12 moles) of sodium carbonate decahidrate are suspended in 350 ml of DMF. 22.3 g (0.24 moles) of 3-amino-1-methoxy-2-propanol are added and the mixture is heated to reflux for 8 hours. It is then filtered and concentrated to dryness. The residue is dissolved in methanol and precipitated with isopropanol. 38 g (Yield: 71.9%) are obtained

### b) N,N'-bis-(2,3-dihydroxypropyl)-5-{3-[N-(2,3-dihydroxypropyl)carbamoyl]-5-methoxyacelamido-2,4,6-triiodobenzoyl-N-(2-hydroxy-3-methoxypropyl)aminoacetyl-N-methylamino}-2,4,6-triiodoisophthalamide.

30 g (0.035 moles) of 39a) and 28.39 g (0.035 moles) of the compound from example la) are dissolved in 180 ml of DMA. 28.6 g (0.1 moles) of Na₂CO₃.10H₂O are added and stirred at room temperature for 20 h. It is filtered and the solution is evaporated to dryness. The residue is dissolved in methanol and precipitated with isopropanol. The crude product obtained is dissolved in (1:1) methanol/water and hydrolysed with 5% NaOH at 45 -50°C. It is then neutralised and evaporated to dryness. The crude product obtained is purified by preparative liquid chromatography. 21 g (Yield: 38.7%) are obtained.

### Example 40

### N,N'-bis-(2,3-dihydroxypropyl)-5-{3-[N-(2,3-dihydroxypropyl) carbamoyl]-5-hydroxyacetyl-N-(2-methoxyethyl)amino-2,4,6-triiodobenzoylaminoacetyl-N-(2-methoxyethyl)amino} -2,4,6-triiodoisophthalamide. (lCJ-2191)

This is prepared by following the general procedure described for the compound from Example 16) and starting from: 25 g (0.017 mol) of the compound from Example 18, 35.1 g (0.092 mol) of trisodium phosphate dodecahydrate and 6.9 g (0.073 mol) of 2-chloro-1-methoxyethane to obtain 20 g of the dimer sought, which is purified by preparative liquid chromatography.

### Example 41

### N,N'-bis-(2,3-dihydroxypropyl)-5-{3-[N-(2-hydroxyethyl)-N-(2,3-dihydroxypropyl)carbamoyl]-5-methoxyacetamido -2,4,6-triiodobenzoyl-N-methylaminoacetyl-N-methylamino} --2,4,6-triiodoisophthalamide. (lCJ-2291)

### a) N-(2-hydroxyethyl)-N-(2,3-dihydroxypropyl)-5-methoxyacetamido-2,4,6-triiodoisophthalamoyl chloride

To a cold solution of 23,37 g (35 mmol) of 5-methoxyacetamido-2,4,6-triiodoisophthaloyl chloride in 100 ml of DMF are slowly added a solution of 4.55 g (45 mmol) of triethylamine and 6.08 g (45 mmol) of 2-hydroxyethyl-2,3-dihydroxypropylamin in 30 ml of DMF. Once the addition has been completed, it is heated to 10-15°C. It is stirred for 2-3 hours and the reaction medium is distributed between 300 ml of ethyl acetate and 400 ml of water. The organic phase is decanted off and the aqueous phase is extracted with ethyl acetate (2x50 ml). The combined organic phases are washed with 5% sodium bicarbonate (1x50 ml) and saturated sodium chloride (1x50 ml). It is dried with anhydrous magnesium sulphate, filtered and the solvent removed under reduced pressure. The slightly coloured solid residue is dried in vacuo with P₂O₅, to obtain 16.6 g (62%) of product.

### b) N,N'-bis-(2,3-dihydroxypropyl)-5-{3-[N-(2-hydroxyethyl) N-(2,3-dihydroxypropyl)carbamoyl]-5-methoxyacetamido-2,4,6-triiodobenzoyl-N-methylaminoacetyl-N-methylamino}-2,4,6-triiodoisophthalamide.

Following the general procedure described in example 1b) and starting with the compound described in 41a) (25 g, 32.6 mmol) and that described in 11b) (25.7 g, 32.6 mmol), 33 g (66%) of a crude product is obtained, which is purified by preparative liquid chromatography.

### Example 42

### N,N'-bis-(2-hydroxyethyl)-N,N'-bis-(2,3-dihydroxypropyl)-5-{3-[N-(2,3-dihydroxypropyl)carbamoyl]-5-acetamido -2,4,6-triiodobenzoyl-N-methylaminoacetyl-N-methylamino}-2,4,6-triiodoisophthalamide. (lCJ-2391)

### a) N,N'-bis-(2-hydroxyethyl)-N,N'-bis-(2,3-dihydroxypropyl)-5-chloroacetyl-N-methylamino-2,4,6-triiodoisophthalamide

103 g (0.15 moles) of the compound from example 3a) are suspended in 309 ml of DMF. 85.8 g (0.3 moles) Na₂CO₃.10H₂O are added and cooled to between 0 and 5°C. A solution of 40.5 g (0.3 moles) of 2-hydroxyethyl-2,3-dihydroxypropylamine in 90 ml of DMF are added, and the mixture is stirred at room temperature for 20 h. The solution is filtered and evaporated to dryness. The residue is dissolved in methanol and precipitated in ethyl ether. 96 g (72.4%) are obtained.

### b) N,N'-bis-(2-hydroxyethyl)-N,N'-bis-(2,3-dihydroxypropyl)-5-methylaminoacetyl-N-methylamino-2,4,6-triiodoisophthalamide

60 g (0.068 moles) of the compound from example 42a) are dissolved in 420 ml of 33% methylamine in ethanol and stirred at room temperature for 2-3 days. The solution is evaporated to dryness and the residue is dissolved in methanol and precipitated from isopropanol. 42 g (Yield: 70.3%) are obtained.

### c) N,N'-bis-(2-hydroxyethyl)-N,N'-bis-(2,3-dihydroxypropyl)-5-{3-[N-(2,3-dihydroxypropyl)carbamoyl]-5-acetamido-2,4,6-triiodobenzoyl-N-methylaminoacetyl-N-methylamino}-2,4,6-triiodoisophthalamide.

35 g (0.04 moles) of the compound from example 42b) and 31 g (0.04 moles) of the compound from example 36b) are dissolved in 210 ml of DMA. 34.3 g (0.12 moles) of Na₂CO₃.10H₂O are added and stirred at room temperature for 20 hours. The mixture is filtered and the solution is evaporated to dryness. The residue is dissolved in methanol and precipitated with isopropanol. The crude product obtained is dissolved in (1:1) methanol/water and hydrolysed with 5% NaOH at 45-50°C. It is then neutralised and evaporated to dryness. The crude product obtained is purified by preparative liquid chromatography.
23 g (Yield: 37.5%) are obtained.

## Claims

1. Non-ionic dimeric compounds derived from aminotriiodo isophthalic acid with the general formula I 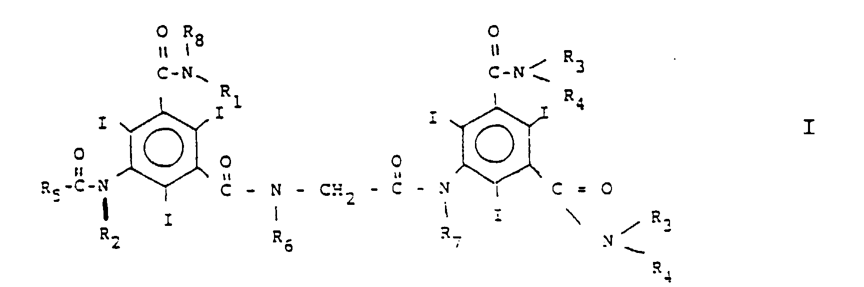 where,
R₁ represents a linear or branched C₂-C₄ polyhydroxylalkyl radical;
R₂ represents hydrogen, methyl, 2-hydroxyethyl, a linear C₂-C₃ polyhydroxyalkyl radical, 2-methoxyethyl or 2-hydroxy-3-methoxypropyl;
R₃ and R₈ are the same or different, and represent hydrogen, methyl or 2-hydroxyethyl;
R₄ represents a linear or branched C₂ to C₄ polyhydroxyalkyl radical;
R₅ represents methyl, hydroxymethyl or methoxymethyl;
R₆ and R₇ are the same or different, and represent hydrogen, methyl, 2-hydroxyethyl 2-methoxyethyl, 2,3-dihydroxypropyl or 2-hydroxy-3-methoxypropyl.
R₂ and R₅ together form a group of the type 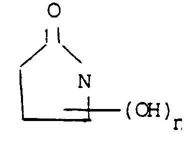 where n can be 0 to 3.

2. N,N'-bis-(2,3-dihydroxypropyl)-N,N'-dimethyl-5-{3-[N-(2,3-dihydroxypropyl)carbamoyl]-5-methoxyacetanido-2,4,6-triiodobenzoylaminoacetamido}-2,4,6-triiodoisophthalamide.

3. N,N'-bis-(2,3-dihydroxypropyl)-N,N'-dimethyl-5-{3-[N-(2,3-dihydroxypropyl)carbamoyl]-5-hydroxyacetamido-2,4,6-triiodobenzoylaminoacetamido}-2,4,6-triiodoisophthalamide.

4. N,N'-bis-(1-hydroxymethyl-2,3-dihydroxypropyl)-5-{3[N-(2,3-dihydroxypropyl)carbamoyl]-5-hydroxyacetamido-2,4,6-triiodobenzoylaminoacetyl-N-methylamino}-2,4;6-triiodoisophthalamide.

5. N,N'-bis-(1-hydroxymethyl-2,3-dihydroxypropyl)-5-{3-[N-(2,3-dihydroxypropyl)carbamoyl]-5-methoxyacetamido-2,4,6-triiodobenzoylaminoacetyl-N-methylamino}-2,4,6-triiodoisophthalamide.

6. N,N'-bis-(2,3-dihydroxypropyl)-N,N'-dimethyl-5-{3-[N-(1-hydroxymethyl-2,3-dihydroxypropyl)carbamoyl]-5-methoxyacetamido-2,4,6-triiodobenzoylaminoacetamido}-2,4,6-triiodoisophthalamide.

7. N,N'-bis-(2,3-dihydroxypropyl)-5-{3-[N-(1-hydroxymethyl-2,3-dihydroxypropyl)carbamoyl]-5-methoxyacetamido-2,4,6-triiodobenzoylaminoacetamido}-2,4,6-triiodoisophthalamide.

8. N,N'-bis-(1-hydroxymethyl-2,3-dihydroxypropyl)-5-{3-[N-(1-hydroxymethyl-2,3-dihydroxypropyl)carbamoyl]-5-methoxyacetamido-2,4,6-triiodobenzoylaminoacetamido}-2,4,6-triiodoisophthalamide.

9. N,N'-bis-(1-hydroxymethyl-2,3-dihydroxypropyl)--5-{3-[N-(2,3-dihydroxypropyl)carbamoyl]-5-hydroxyacetamido-2,4,6-triiodobenzoyl-N-methylaminoacetyl-N-methylamino}-2,4,6-triiodoisophthalamide.

10. N,N'-bis-(1-hydroxymethyl-2,3-dihydroxypropyl)-5-{3-[N-(2,3-dihydroxypropyl)carbamoyl]-5-methoxyacetamido-2,4,6-triiodobenzoyl-N-methylaminoacetyl-N-methylamino}-2,4,6-triiodoisophthalamide.

11. N,N'-bis-(1-hydroxymethyl-2,3-dihydroxypropyl)-5-{3-[N-1-hydroxymethyl-2,3-dihydroxypropyl)carbamoyl]-5-methoxyacetamido-2,4,6-triiodobenzoyl-N-methylaminoacetyl-N-methylanino}-2,4,6-triiodoisophthalamide.

12. N,N'-bis-(2,3-dihydroxypropyl)-5-{3-[N-(2,3-dihydroxypropyl)carbamoyl]-5-hydroxyacetamido-2,4,6-triiodobenzoyl-N-methylaminoacetyl-N-methylamino)-2,4,6-triiodoisophthalamide.

13. N,N'-bis-(2,3-dihydroxypropyl)-5-{3-[N-2.3-dihydroxypropyl)carbamoyl]-5-methoxyacetamido-2,4,6-triiodobenzoyl-N-methylaminoacetyl-N-methylamino}-2,4,6-triiodo isophthalamide.

14. N,N'-bis-(2,3-dihydroxypropyl)-5-{3-[N-(2,3-dihydroxypropyl)carbamoyl]-5-hydroxyacetamido-2,4,6-triiodobenzoyl-N-(2,3-dihydroxypropyl)aminoacetamido}-2,4,6-triiodoisophthalamide.

15. N,N'-bis-(2-hydroxyethyl)-5-{3-[N-(2,3-dihydroxypropyl)carbamoyl]-5-hydroxyacetamido-2,4,6-triiodobenzoyl-N-(2-hydroxyethyl)aminoacetamido}-2,4,6-triiodoisophthalamide.

16. N,N'-bis-(2,3-dihydroxypropyl)-5-{3-[N-2,3-dihydroxypropyl)carbamoyl]-5-methoxyacetamido-2,4,6-triiodobenzoyl-N-(2,3-dihydroxypropyl)aminoacetamido}-2,4,6-triiodoisophthalamide.

17. N,N'-bis-(2-hydroxyethyl)-5-{3-[N-(2,3-dihydroxypropyl)carbamoyl]-5-hydroxyacetyl-N-(2-hydroxyethyl)amino-2,4,6-triiodobenzoyl-N-(2-hydroxyethyl)aminoacetyl-N-(2-hydroxyethyl)amino}-2,4,6-triiodoisophthalamide.

18. N,N'-bis-(2-hydroxyethyl)-5-{3-[N-(2,3-dihydroxypropyl)carbamoyl]-5-methoxyacetamido-2,4,6-triiodobenzoyl-N-(2-hydroxyethyl)aminoacetamido}-2,4,6-triiodoisophthalamide.

19. N,N'-bis-(2,3-dihydroxypropyl)-5-{3-[N-(2,3-dihydroxypropyl)carbamoyl]-5-hydroxyacetamido-2,4,6-triiodobenzoylaminoacetamido}-2,4,6-triiodoisophthalamide.

20. N,N'-bis-(1-hydroxymethyl-2,3-dihydroxypropyl)-5-{3-[N-(2,3-dihydroxypropyl)carbamoyl]-2,4,6-triiodobenzoylaminoacetamido}-2,4,6-triiodoisophthalamide.

21. N,N'-bis-(1-hydroxymethyl-2,3-dihydroxypropyl)-5-{3-[N-(2,3-dihydroxypropyl)carbamoyl]-5-methoxyacetamido-2,4,6-triiodobenzoylaminoacetamido}-2,4,6-triiodoisophthalamide.

22. N,N'-bis-(1-hydroxymethyl-2,3-dihydroxypropyl) -5-{3-[N-(2,3-dihydroxypropyl)carbamoyl]-5-methoxyacetamido-2,4,6-triiodobenzoyl-N-methylaminoacetamido}-2,4,6-triiodoisophthalamide.

23. N,N'-bis-(1-hydroxymethyl-2,3-dihydroxypropyl)-5-{3-[N-(2,3-dihydroxypropyl)carbamoyl]-5-methoxyacetamido-2,4,6-triiodobenzoyl-N-(2-hydroxyethyl)aminoacetamido}-2,4,6 triiodoisophthalamide.

24. N,N'-bis-(2,3-dihydroxypropyl)-5-{3-[N-(2,3-dihydroxypropyl)carbamoyl]-5-acetamido-2,4,6-triiodobenzoyl-N-(2,3-dihydroxypropyl)aminoacetyl-N-methylamino}-2,4,6-triiodoisophthalamide.

25. N,N'-bis-(2,3-dihydroxypropyl)-N,N'-dimethyl-5-{3-[N-(2,3-dihydroxypropyl)carbamoyl]-5-hydroxyacetamido-2,4,6-triiodobenzoyl-N-methylaminoacetyl-N-methylamino}-2-4-6-triiodoisophthalamide.

26. N,N'-bis-(2,3-dihydroxypropyl)-N,N'-dimethyl-5-{3-[N-(2,3-dihydroxypropyl)carbamoyl]-5-methoxyacetamido-2,4,6-triiodobenzoyl-N-methylaminoacetyl-N-methylamino}-2,4,6-triiodoisophthalamide.

27. N,N'-bis-(2,3-dihydroxypropyl)-N,N'-dimethyl-5-{3-[N-(2,3-dihydroxypropyl)carbamoyl]-5-hydroxyacetamido-2,4,6-triiodobenzoylaminoacetyl-N-methylamino}-2,4,6-triiodo isophthalamide.

28. N,N'-bis-(2,3-dihydroxypropyl)-N,N'-dimethyl-5-{3-[N-(2,3-dihydroxypropyl)carbamoyl]-5-methoxyacetamido-2,4,6-triiodobenzoylaminoacetyl-N-methylamino}-2,4,6-triiodo isophthalamide.

29. N,N'-bis-(2,3-dihydroxypropyl)-5-{3-[N-(2,3-dihydroxypropyl)carbamoyl]-5-methoxyacetamido-2,4,6-triiodobenzoylaminoacetyl-N-methylamino}-2,4,6-triiodoisophthalamide.

30. N,N,N',N'-tetrakis-(2-hydroxyethyl)-5-{3-[N-(2,3-dihydroxypropyl)carbamoyl]-5-hydroxyacetamido-2,4,6-triiodobenzoyl-N-methylaminoacetyl-N-methylamino}-2,4,6-triiodoisophthalamide.

31. N,N,N',N'-tetrakis-(2-hydroxyethyl)-5-{3-[N-(2,3-dihydroxypropyl)carbamoyl]-5-methoxyacetamido-2,4,6-triiodobenzoyl-N-methylaminoacetyl-N-methylamino}-2,4,6-triiodoisophthalamide.

32. N,N,N',N'-tetrakis-(2-hydroxyethyl)-5-{3-[N-(2,3-dihydroxypropyl)carbamoyl]-5-methoxyacetamido-2,4,6-triiodobenzoylaminoacetamido}-2,4,6-triiodoisophthalamide.

33. N,N'-bis-(2-hydroxyethyl)-5-{3-[N-(2,3-dihydroxypropyl)carbamoyl]-5-methoxyacetyl-N-(2-hydroxyethyl)amino-2,4,6-triiodobenzoyl-N-(2-hydroxyethyl)aminoacetyl-N-(2-hydroxyethyl)amino}-2,4,6-triiodoisophthalamide.

34. N,N'-bis-(2,3-dihydroxypropyl)-5-{3-[N-(2,3-dihydroxypropyl)carbamoyl]-5-methoxyacetyl-N-(2-hydroxyethyl)amino-2,4,6-triiodobenzoyl-N-(2-hydroxyethyl)aninoacetyl-N-(2-hydroxyethyl)amino}-2,4,6-triiodoisophthalamide.

35. N,N'-bis-(2,3-dihydroxypropyl)-5-{3-[N-(2,3-dihydroxypropyl)carbamoyl]-5-hydroxyacetyl-N-(2-hydroxyethyl)amino-2,4,6-triiodobenzoylaminoacetyl-N-(2-hydroxyethyl)amino}-2,4,6-triiodoisophthalamide.

36. N,N'-bis-(2,3-dihydroxpropyl)-5-{3-[N-(2,3-dihydroxypropyl)carbamoyl]-5-methoxyacetyl-N-(2-hydroxyethyl) amino-2,4,6-triiodobenzoyl-N-methylaminoacetyl-N-(2-hydroxyethyl)amino}-2,4,6-triiodoisophthalamide.

37. N,N-bis-(2,3-dihydroxypropyl)-5-{3-[N-(2,3-dihydroxypropyl)carbamoyl]-5-acetamido-2,4,6-triiodobenzoyl-N-(2,3-dihydroxypropyl)aminoacetamido}-2,4,6-triiodoisophthalamide.

38. N,N'-bis-(2,3-dihydroxypropyl)-5-{3-[N-(2,3-dihydroxypropyl)carbamoyl-5-acetamido-2,4,6-triiodobenzoyl-N-methylacetyl-N-methylamino}-2,4,6-triiodoisophthalamide.

39. N,N'-bis-(1-hydroxymethyl-2,3-dihydroxypropyl)-5-{3-[N-(2,3-dihydroxypropyl)carbamoyl]-5-acetamido-2,4,6-triiodobenzoyl-N-methylacetyl-N-methylanino}-2,4,6-triiodoisophthalamide.

40. N,N'-bis-(2,3-dihydroxypropyl)-5-{3-[N-(2,3-dihydroxypropyl)carbamoyl]-5-methoxyacetamido-2,4,6-triiodobenzoyl-N-(2-hydroxy-3-methoxypropyl)aminoacetyl-N-methylamino}-2,4,6-triiodoisophthalamide.

41. N,N'-bis-(2,3-dihydroxypropyl)-5-{3-[N-(2,3-dihydroxypropyl)carbamoyl]-5-hydroxyacetyl-N-(2-methoxyethyl)amino-2,4,6-triiodobenzoylaminoacetyl-N-(2-methoxyethyl)amino}-2,4,6-triiodoisophthalamide.

42. N,N'-bis-(2,3-dihydroxypropyl)-5-{3-[N-(2-hydroxyethyl)-N-(2,3-dihydroxypropyl)carbamoyl]-5-methoxyacetamido-2,4,6-triiodobenzoyl-N-methylaminoacetyl-N-methylamino}-2,4,6-triiodoisophthalamide.

43. N,N'-bis-(2-hydroxyethyl)-N,N'-bis-(2,3-dihydroxypropyl)-5-{3-[N-(2,3-dihydroxypropyl)carbamoyl]-5-acetamido-2,4,6-triiodobenzoyl-N-methylaminoacetyl-N-methylamino}-2,4,6-triiodoisophthalamide.

44. Galenic compositions that include the compounds of general formula I, defined in Claim 1, in concentrations of between 100 and 400 mg of I/ml, and incorporating pH stabilisers such as the buffer Tris (= tris(hydroxymethyl)aminometane) and its salts, phosphates, citrates, and hydrogen carbonates, also sterile apyrogenic water, and may also incorporate complexing agents for heavy metals, for example, the sodium and/or calcium salts of ethylenediaminetetracetic acid, and other chelating agents which are pharmaceutically acceptable.

45. Method for the preparation of compounds with the general formula I, defined in Claim 1, characterised by being formed by reacting a compound with the general formula II 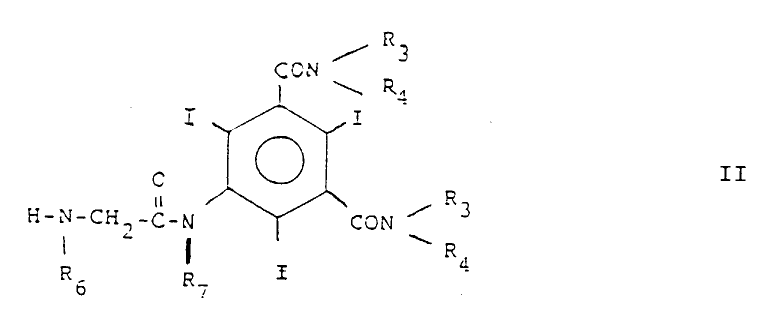 with an acid chloride with the formula III 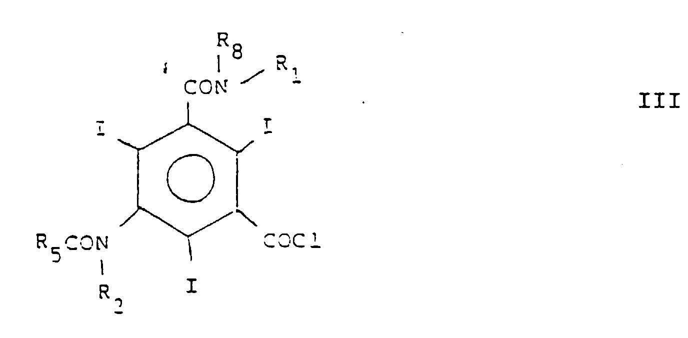 in the presence of a basic catalyst, the radicals R₁ to R₈ having the significance indicated previously; during the reaction, the -OH groups are protected as acetates or, when the -OH groups are vicinal, as dioxolanes.

46. A method for the preparation of compounds with the general formula I by means of an N-alkylation reaction using reagents 2-hydroxyethyl, 2-methoxyethyl, 2-hydroxy-3methoxypropyl and 2,3-dihydroxypropyl chlorides in an alkaline medium, with the compounds of formula I in which R₂ and R₇ are hydrogen.

## Patentansprüche

1. Nicht-ionische dimere, von Aminotrijodisophthalsäure abgeleitete Verbindungen nach der allgemeinen Formel I 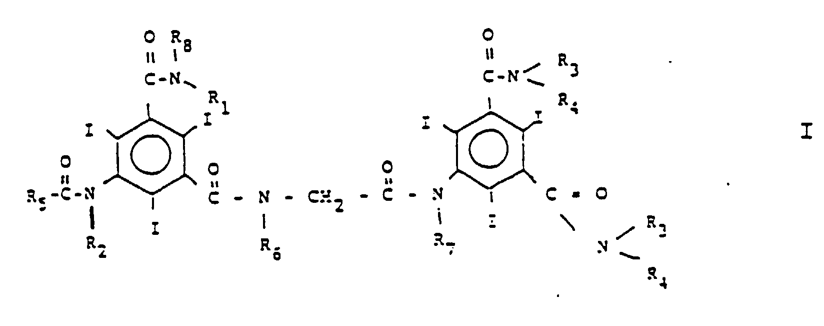 worin
R₁ einen linearen oder verzweigten C₂-C₄ Polyhydroxyalkylrest darstellt,
R₂ Wasserstoff, Methyl, 2-Hydroxyethyl, einen linearen C₂-C₃ Polyhydroxyalkylrest, 2-Methoxyethyl oder 2-Hydroxy-3-methoxypropyl darstellt,
R₃ und R₈ gleich oder unterschiedlich sind und Wasserstoff, Methyl oder 2-Hydroxyethyl darstellen,
R₄ einen linearen oder verzweigten C₂ bis C₄ Polyhydroxyalkylrest darstellt,
R₅ Methyl, Hydroxymethyl oder Methoxymethyl darstellt,
R₆ und R₇ gleich oder unterschiedlich sind und Wasserstoff, Methyl, 2-Hydroxyethyl, 2-Methoxyethyl, 2,3-Dihydroxypropyl oder 2-Hydroxy-3-methoxypropyl darstellen,
R₂ und R₅ zusammen eine Gruppe vom Typ 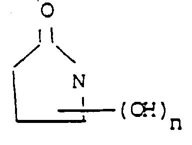 bilden, wobei n 0 bis 3 sein kann.

2. N,N'-bis-(2,3-dihydroxypropyl)-N,N'-dimethyl-5-{3-[N-(2,3-dihydroxypropyl)carbamoyl]-5-methoxyacetamido-2,4,6-trijodbenzoylaminoacetamido}-2,4,6-trijodisophthalamid.

3. N,N'-bis-(2,3-dihydroxypropyl)-N,N'-dimethyl-5-{3-[N-(2,3-dihydroxypropyl)carbamoyl]-5-hydroxyacetamido-2,4,6-trijodbenzoylaminoacetamido}-2,4,6-trijodisophthalamid.

4. N,N'-bis-(1-hydroxymethyl-2,3-dihydroxypropyl)-5-{3-[N-(2,3-dihydroxypropyl)carbamoyl]-5-hydroxyacetamido-2,4,6-trijodbenzoylaminoacetyl-N-methylamino}-2,4,6-trijodisophthalamid.

5. N,N'-bis-(1-hydroxymethyl-2,3-dihydroxypropyl)-5-{3-[N-(2,3-dihydroxypropyl)carbamoyl]-5-methoxyacetamido-2,4,6-trijodbenzoylaminoacetyl-N-methylamino}-2,4,6-trijodisophthalamid.

6. N,N'-bis-(2,3-dihydroxypropyl)-N,N'-dimethyl-5-{3-[N-(1-hydroxymethyl-2,3-dihydroxypropyl)carbamoyl]-5-methoxyacetamido-2,4,6-trijodbenzoylaminoacetamido}-2,4,6-trijodisophthalamid.

7. N,N'-bis-(2,3-dihydroxypropyl)-5-{3-[N-(1-hydroxymethyl-2,3-dihydroxypropyl)carbamoyl]-5-methoxyacetamido-2,4,6-trijodbenzoylaminoacetamido}-2,4,6-trijodisophthalamid.

8. N,N'-bis-(1-hydroxymethyl-2,3-dihydroxypropyl)-5-{3-[N-(1-hydroxymethyl-2,3-dihydroxypropyl)carbamoyl]-5-methoxyacetamido-2,4,6-trijodbenzoylaminoacetamido}-2,4,6-trijodisophthalamid.

9. N,N'-bis-(1-hydroxymethyl-2,3-dihydroxypropyl)-5-{3-[N-(2,3-dihydroxypropyl)carbamoyl]-5-hydroxyacetamido-2,4,6-trijodbenzoyl-N-methylaminoacetyl-N-methylamino}-2,4,6-trijodisophthalamid.

10. N,N'-bis-(1-hydroxymethyl-2,3-dihydroxypropyl)-5-{3-[N-(2,3-dihydroxypropyl)carbamoyl]-5-methoxyacetamido-2,4,6-trijodbenzoyl-N-methylaminoacetyl-N-methylamino}-2,4,6-trijodisophthalamid.

11. N,N'-bis-(1-hydroxymethyl-2,3-dihydroxypropyl)-5-{3-[N-(1-hydroxymethyl-2,3-dihydroxypropyl)carbamoyl]-5-methoxyacetamido-2,4,6-trijodbenzoyl-N-methylaminoacetyl-N-methylamino}-2,4,6-trijodisophthalamid.

12. N,N'-bis-(2,3-dihydroxypropyl)-5-{3-[N-(2,3-dihydroxypropyl)carbamoyl]-5-hydroxyacetamido-2,4,6-trijodbenzoyl-N-methylaminoacetyl-N-methylamino}-2,4,6-trijodisophthalamid.

13. N,N'-bis-(2,3-dihydroxypropyl)-5-{3-[N-(2,3-dihydroxypropyl) carbamoyl]-5-methoxyacetamido-2,4,6-trijodbenzoyl-N-methylaminoacetyl-N-methylamino}-2,4,6-trijodisophthalamid.

14. N,N'-bis-(2,3-dihydroxypropyl)-5-{3-[N-(2,3-dihydroxypropyl) carbamoyl]-5-hydroxyacetamido-2,4,6-trijodbenzoyl-N-(2,3-dihydroxypropyl) aminoacetamido}-2,4,6-trijodisophthalamid.

15. N,N'-bis-(2-hydroxyethyl)-5-{3-[N-(2,3-dihydroxypropyl) carbamoyl]-5-hydroxyacetamido-2,4,6-trijodbenzoyl-N-(2-hydroxyethyl)aminoacetamido}-2,4,6-trijodisophthalamid.

16. N,N'-bis-(2,3-dihydroxypropyl)-5-{3-[N-(2,3-dihydroxypropyl) carbamoyl]-5-methoxyacetamido-2,4,6-trijodbenzoyl-N-(2,3-dihydroxypropyl)aminoacetamido}-2,4,6-trijodisophthalamid.

17. N,N'-bis-(2-hydroxyethyl)-5-{3-[N-(2,3-dihydroxypropyl) carbamoyl]-5-hydroxyacetyl-N-(2-hydroxyethyl)amino-2,4,6-trijodbenzoyl-N-(2-hydroxyethyl)aminoacetyl-N-(2-hydroxyethyl)amino}-2,4,6-trijodisophthalamid.

18. N,N'-bis-(2-hydroxyethyl)-5-{3-[N-(2,3-dihydroxypropyl) carbamoyl]-5-methoxyacetamido-2,4,6-trijodbenzoyl-N-(2-hydroxyethyl)aminoacetamido}-2,4,6-trijodisophthalamid.

19. N,N'-bis-(2,3-dihydroxypropyl)-5-{3-[N-(2,3-dihydroxypropyl) carbamoyl]-5-hydroxyacetamido-2,4,6-trijodbenzoylaminoacetamido}-2,4,6-trijodisophthalamid.

20. N,N'-bis-(1-hydroxymethyl-2,3-dihydroxypropyl)-5-{3-[N-(2,3-dihydroxypropyl)carbamoyl]-5-hydroxyacetamido-2,4,6-trijodbenzoylaminoacetamido}-2,4,6-trijodisophthalamid.

21. N,N'-bis-(1-hydroxymethyl-2,3-dihydroxypropyl)-5-{3-[N-(2,3-dihydroxypropyl)carbamoyl]-5-methoxyacetamido-2,4,6-trijodbenzoylaminoacetamido}-2,4,6-trijodisophthalamid.

22. N,N'-bis-(1-hydroxymethyl-2,3-dihydroxypropyl)-5-{3-[N-(2,3-dihydroxypropyl)carbamoyl]-5-methoxyacetamido-2,4,6-trijodbenzoyl-N-methylaminoacetamido}-2,4,6-trijodisophthalamid.

23. N,N'-bis-(1-hydroxymethyl-2,3-dihydroxypropyl)-5-{3-[N-(2,3-dihydroxypropyl)carbamoyl]-5-methoxyacetamido-2,4,6-trijodbenzoyl-N-(2-hydroxyethyl)aminoacetamido}-2,4,6-trijodisophthalamid.

24. N,N'-bis-(2,3-dihydroxypropyl)-5-{3-[N-(2,3-dihydroxypropyl) carbamoyl]-5-acetamido-2,4,6-trijodbenzoyl-N-(2,3-dihydroxypropyl)aminoacetyl-N-methylamino}-2,4,6-trijodisophthalamid.

25. N,N'-bis-(2,3-dihydroxypropyl)-N,N'-dimethyl-5-{3-[N-(2,3-dihydroxypropyl)carbamoyl]-5-hydroxyacetamido-2,4,6-trijodbenzoyl-N-methylaminoacetyl-N-methylamino}-2,4,6-trijodisophthalamid.

26. N,N'-bis-(2,3-dihydroxypropyl)-N,N'-dimethyl-5-{3-[N-(2,3-dihydroxypropyl)carbamoyl]-5-methoxyacetamido-2,4,6-trijodbenzoyl-N-methylaminoacetyl-N-methylamino}-2,4,6-trijodisophthalamid.

27. N,N'-bis-(2,3-dihydroxypropyl)-N,N'-dimethyl-5-{3-[N-(2,3-dihydroxypropyl)carbamoyl]-5-hydroxyacetamido-2,4,6-trijodbenzoylaminoacetyl-N-methylamino}-2,4,6-trijodisophthalamid.

28. N,N'-bis-(2,3-dihydroxypropyl)-N,N'-dimethyl-5-{3-[N-(2,3-dihydroxypropyl)carbamoyl]-5-methoxyacetamido-2,4,6-trijodbenzoylaminoacetyl-N-methylamino}-2,4,6-trijodisophthalamid.

29. N,N'-bis-(2,3-dihydroxypropyl)-5-{3-[N-(2,3-dihydroxypropyl) carbamoyl]-5-methoxyacetamido-2,4,6-trijodbenzoylaminoacetyl-N-methylamino}-2,4,6-trijodisophthalamid.

30. N,N,N',N'-tetrakis-(2-hydroxyethyl)-5-{3-[N-(2,3-dihydroxypropyl)carbamoyl]-5-hydroxyacetamido-2,4,6-trijodbenzoyl-N-methylaminoacetyl-N-methylamino}-2,4,6-trijodisophthalamid.

31. N,N,N',N'-tetrakis-(2-hydroxyethyl)-5-{3-[N-(2,3-dihydroxypropyl)carbamoyl]-5-methoxyacetamido-2,4,6-trijodbenzoyl-N-methylaminoacetyl-N-methylamino}-2,4,6-trijodisophthalamid.

32. N,N,N',N'-tetrakis-(2-hydroxyethyl)-5-{3-[N-(2,3-dihydroxypropyl)carbamoyl]-5-methoxyacetamido-2,4,6-trijodbenzoylaminoacetamido}-2,4,6-trijodisophthalamid.

33. N,N'-bis-(2-hydroxyethyl)-5-{3-[N-(2,3-dihydroxypropyl) carbamoyl]-5-methoxyacetyl-N-(2-hydroxyethyl)amino-2,4,6-trijodbenzoyl-N-(2-hydroxyethyl)aminoacetyl-N-(2-hydroxyethyl)amino}-2,4,6-trijodisophthalamid.

34. N,N'-bis-(2,3-dihydroxypropyl)-5-{3-[N-(2,3-dihydroxypropyl) carbamoyl]-5-methoxyacetyl-N-(2-hydroxyethyl)amino-2,4,6-trijodbenzoyl-N-(2-hydroxyethyl)aminoacetyl-N-(2-hydroxyethyl)amino}-2,4,6-trijodisophthalamid.

35. N,N'-bis-(2,3-dihydroxypropyl)-5-{3-[N-(2,3-dihydroxypropyl) carbamoyl]-5-hydroxyacetyl-N-(2-hydroxyethyl)amino-2,4,6-trijodbenzoylaminoacetyl-N-(2-hydroxyethyl)amino}-2,4,6-trijodisophthalamid.

36. N,N'-bis-(2,3-dihydroxypropyl)-5-{3-[N-(2,3-dihydroxypropyl) carbamoyl]-5-methoxyacetyl-N-(2-hydroxyethyl)amino-2,4,6-trijodbenzoyl-N-methylaminoacetyl-N-(2-hydroxyethyl)amino}-2,4,6-trijodisophthalamid.

37. N,N-bis-(2,3-dihydroxypropyl)-5-{3-[N-(2,3-dihydroxypropyl) carbamoyl]-5-acetamido-2,4,6-trijodbenzoyl-N-(2,3-dihydroxypropyl)aminoacetamido}-2,4,6-trijodisophthalamid.

38. N,N'-bis-(2,3-dihydroxypropyl)-5-{3-[N-(2,3-dihydroxypropyl) carbamoyl]-5-acetamido-2,4,6-trijodbenzoyl-N-methylacetyl-N-methylamino}-2,4,6-trijodisophthalamid.

39. N,N'-bis-(1-hydroxymethyl-2,3-dihydroxypropyl)-5-{3-[N-(2,3-dihydroxypropyl)carbamoyl]-5-acetamido-2,4,6-trijodbenzoyl-N-methylacetyl-N-methylamino}-2,4,6-trijodisophthalamid.

40. N,N'-bis-(2,3-dihydroxypropyl)-5-{3-[N-(2,3-dihydroxypropyl) carbamoyl]-5-methoxyacetamido-2,4,6-trijodbenzoyl-N-(2-hydroxy-3-methoxypropyl)aminoacetyl-N-methylamino}-2,4,6-trijodisophthalamid.

41. N,N'-bis-(2,3-dihydroxypropyl)-5-{3-[N-(2,3-dihydroxypropyl) carbamoyl]-5-hydroxyacetyl-N-(2-nethoxyethyl)amino-2,4,6-trijodbenzoylaminoacetyl-N-(2-methoxyethyl)amino}-2,4,6-trijodisophthalamid.

42. N,N'-bis-(2,3-dihydroxypropyl)-5-{3-[N-(2-hydroxyethyl)-N-(2,3-dihydroxypropyl)carbamoyl]-5-nethoxyacetamido-2,4,6-trijodbenzoyl-N-methylaminoacetyl-N-methylamino}-2,4,6-trijodisophthalamid.

43. N,N'-bis-(2-hydroxyethyl)-N,N'-bis-(2,3-dihydroxypropyl)-5-{3-[N-(2,3-dihydroxypropyl)carbamoyl]-5-acetamido-2,4,6-trijodbenzoyl-N-methylaminoacetyl-N-methylamino}-2,4,6-trijodisophthalamid.

44. Galenische Zusammensetzungen, die die Verbindungen nach der allgemeinen, in Anspruch 1 definierten Formel I in Konzentrationen zwischen 100 und 400 mg I/ml umfassen, und pH-Stabilisatoren enthalten, wie etwa den Puffer Tris (=Tris(hydroxymethyl)aminomethan) und dessen Salze, Phosphate, Citrate und Hydrogencarbonate, sowie steriles und pyrogenfreies Wasser, und auch Komplexbildner für Schwermetalle, wie etwa die Natrium- und/oder Calziumsalze von Ethylendiamintetraessigsäure, und andere pharmazeutisch verträgliche Chelatbildner enthalten können.

45. Verfahren zur Herstellung von Verbindungen nach der allgemeinen, in Anspruch 1 definierten Formel I, dadurch gekennzeichnet, daß sie gebildet werden durch Umsetzen einer Verbindung nach der allgemeinen Formel II 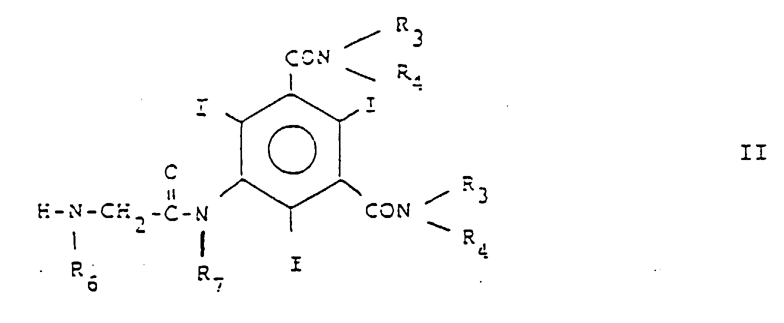 mit einem Säurechlorid nach Formel III 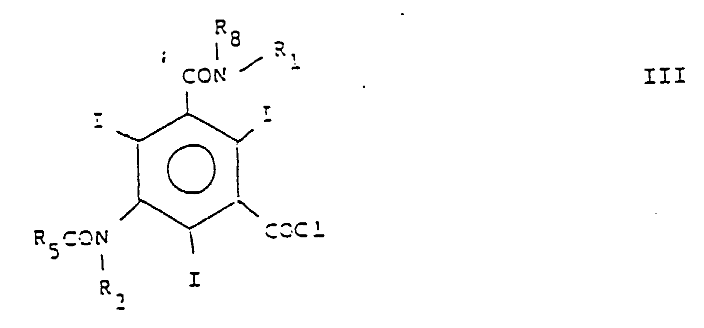 in Gegenwart eines basischen Katalysators, wobei die Reste R₁ bis R₈ die zuvor angegebene Bedeutung haben; wobei während der Reaktion die OH-Gruppen als Acetate oder, wenn die OH-Gruppen vicinal sind, als Dioxolane geschützt werden.

46. Verfahren zur Herstellung von Verbindungen nach der allgemeinen Formel I mittels einer N-Alkylierungsreakticn unter Verwendung von Reagentien 2-Hydroxyethyl-, 2-Methoxyethyl-, 2-Hydroxy-3-methoxypropyl- und 2,3-Dihydroxypropylchlorid in einem alkalischen Medium mit den Verbindungen nach Formel I, wobei R₂ und R₇ Wasserstoff sind.

## Revendications

1. Composés dimériques non ioniques dérivés de l'acide aminotriiodoisophtalique de formule générale I : 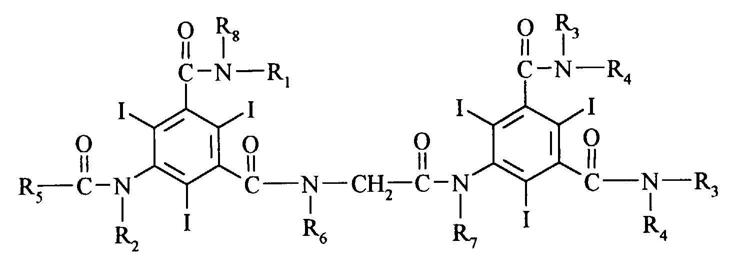 dans laquelle,
R₁ représente un radical polyhydroxyalkyle en C₂-C₄ linéaire ou ramifié;
R₂ représente l'hydrogène, un radical méthyle, 2-hydroxyéthyle, polyhydroxyalkyle en C₂-C₃ linéaire, ou 2-hydroxy-3-méthoxypropyle;
R₃ et R₈ sont identiques ou différents, et représentent l'hydrogène, un radical méthyle ou 2-hydroxyéthyle;
R₄ représente un radical polyhydroxyalkyle en C₂-C₄ linéaire ou ramifié;
R₅ représente un radical méthyle, hydroxyméthyle ou méthoxyméthyle;
R₆ et R₇ sont identiques ou différents, et représentent l'hydrogène, un radical méthyle, 2-hydroxyéthyle, 2-méthoxyéthyle, 2,3-dihydroxypropyle ou 2-hydroxy-3-méthoxypropyle;
R₂ et R₅ forment ensemble un groupe de type 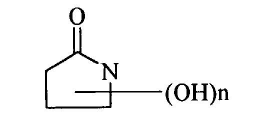 dans lequel n peut être compris entre 0 et 3.

2. N,N'-bis-(2,3-dihydroxypropyl)-N,N'-diméthyl-5-{3-[N-(2,3-dihydroxypropyl)carbamoyl]-5-méthoxyacétamido-2,4,6-triiodobenzoylaminoacétamido}-2,4,6-triiodoisophtalamide.

3. N,N'-bis-(2,3-dihydroxypropyl)-N,N'-diméthyl-5-{3-[N-(2,3-dihydroxypropyl)carbamoyl]-5-hydroxyacétamido-2,4,6-triiodobenzoylaminoacétamido}-2,4,6-triiodoisophtalamide.

4. N,N'-bis-(1-hydroxyméthyl-2,3-dihydroxypropyl)-5-{3-[N-(2,3-dihydroxypropyl)carbamoyl]-5-hydroxyacétamido-2,4,6-triiodobenzoylaminoacétyl-N-méthylamino}-2,4,6-triiodoisophtalamide.

5. N,N'-bis-(1-hydroxyméthyl-2,3-dihydroxypropyl)-5-{3-[N-(2,3-dihydroxypropyl)carbamoyl]-5-méthoxyacétamido-2,4,6-triiodobenzoylaminoacétyl-N-méthylamino}-2,4,6-triiodoisophtalamide.

6. N,N'-bis-(2,3-dihydroxypropyl)-N,N'-diméthyl-5-{3-[N-(1-hydroxyméthyl-2,3-dihydroxypropyl)carbamoyl]-5-méthoxyacétamido-2,4,6-triiodobenzoylaminoacétamido}-2,4,6-triiodoisophtalamide.

7. N,N'-bis-(2,3-dihydroxypropyl)-5-{3-[N-(1-hydroxyméthyl-2,3-dihydroxypropyl)carbamoyl]-5-méthoxyacétamido-2,4,6-triiodobenzoylaminoacétamido}-2,4,6-triiodoisophtalamide.

8. N,N'-bis-(1-hydroxyméthyl-2,3-dihydroxypropyl)-5-{3-[N-(1-hydroxyméthyl-2,3-dihydroxypropyl)carbamoyl]-5-méthoxyacétamido-2,4,6-triiodobenzoylaminoacétamido}-2,4,6-triiodoisophtalamide.

9. N,N'-bis-(1-hydroxyméthyl-2,3-dihydroxypropyl)-5-{3-[N-(2,3-dihydroxypropyl)carbamoyl]-5-hydroxyacétamido-2,4,6-triiodobenzoyl-N-méthylaminoacétyl-N-méthylamino}-2,4,6-triiodoisophtalamide.

10. N,N'-bis-(1-hydroxyméthyl-2,3-dihydroxypropyl)-5-{3-[N-(2,3-dihydroxypropyl)carbamoyl]-5-méthoxyacétamido-2,4,6-triiodobenzoyl-N-méthylaminoacétyl-N-méthylamino}-2,4,6-triiodoisophtalamide.

11. N,N'-bis-(1-hydroxyméthyl-2,3-dihydroxypropyl)-5-{3-[N-(1-hydroxyméthyl-2,3-dihydroxypropyl)carbamoyl]-5-méthoxyacétamido-2,4,6-triiodobenzoyl-N-méthylaminoacétyl-N-méthylamino}-2,4,6-triiodoisophtalamide.

12. N,N'-bis-(2,3-dihydroxypropyl)-5-{3-[N-(2,3-dihydroxypropyl)carbamoyl]-5-hydroxyacétamido-2,4,6-triiodobenzoyl-N-méthylaminoacétyl-N-méthylamino}-2,4,6-triiodoisophtalamide.

13. N,N'-bis-(2,3-dihydroxypropyl)-5-{3-[N-(2,3-dihydroxypropyl)carbamoyl]-5-méthoxyacétamido-2,4,6-triiodobenzoyl-N-méthylaminoacétyl-N-méthylamino}-2,4,6-triiodoisophtalamide.

14. N,N'-bis-(2,3-dihydroxypropyl)-5-{3-[N-(2,3-dihydroxypropyl)carbamoyl]-5-hydroxyacétamido-2,4,6-triiodobenzoyl-N-(2,3-dihydroxypropyl)aminoacétamido}-2,4,6-triiodoisophtalamide.

15. N,N'-bis-(2-hydroxyéthyl)-5-{3-[N-(2,3-dihydroxypropyl)carbamoyl]-5-hydroxyacétamido-2,4,6-triiodobenzoyl-N-(2-hydroxyéthyl)aminoacétamido}-2,4,6-triiodoisophtalamide.

16. N,N'-bis-(2,3-dihydroxypropyl)-5-{3-[N-(2,3-dihydroxypropyl)carbamoyl]-5-méthoxyacétamido-2,4,6-triiodobenzoyl-N-(2,3-dihydroxypropyl)aminoacétamido}-2,4,6-triiodoisophtalamide.

17. N,N'-bis-(2-hydroxyéthyl)-5-{3-[N-(2,3-dihydroxypropyl)carbamoyl]-5-hydroxyacétyl-N-(2-hydroxyéthyl)amino-2,4,6-triiodobenzoyl-N-(2-hydroxyéthyl)amineacétyl-N-(2-hydroxyéthyl)amino}-2,4,6-triiodoisophtalamide.

18. N,N'-bis-(2-hydroxyéthyl)-5-{3-[N-(2,3-dihydroxypropyl)carbamoyl]-5-méthoxyacétamido-2,4,6-triiodobenzoyl-N-(2-hydroxyéthyl)aminoacétamido}-2,4,6-triiodoisophtalamide.

19. N,N'-bis-(2,3-dihydroxypropyl)-5-{3-[N-(2,3-dihydroxypropyl)carbamoyl]-5-hydroxyacétamido-2,4,6-triiodobenzoylaminoacétamido}-2,4,6-triiodoisophtalamide.

20. N,N'-bis-(1-hydroxyméthyl-2,3-dihydroxypropyl)-5-{3-[N-(2,3-dihydroxypropyl)carbamoyl]-5-hydroxyacétamido-2,4,6-triiodobenzoylaminoacétamido}-2,4,6-triiodoisophtalamide.

21. N,N'-bis-(1-hydroxyméthyl-2,3-dihydroxypropyl)-5-{3-[N-(2,3-dihydroxypropyl)carbamoyl]-5-méthoxyacétamido-2,4,6-triiodobenzoylaminoacétamido}-2,4,6-triiodoisophtalamide.

22. N,N'-bis-(1-hydroxyméthyl-2,3-dihydroxypropyl)-5-{3-[N-(2,3-dihydroxypropyl)carbamoyl]-5-méthoxyacétamido-2,4,6-triiodobenzoyl-N-méthylaminoacétamido}-2,4,6-triiodoisophtalamide.

23. N,N'-bis-(1-hydroxyméthyl-2,3-dihydroxypropyl)-5-{3-[N-(2,3-dihydroxypropyl)carbamoyl]-5-méthoxyacétamido-2,4,6-triiodobenzoyl-N-(2-hydroxyéthyl)aminoacétamido}-2,4,6-triiodoisophtalamide.

24. N,N'-bis-(2,3-dihydroxypropyl)-5-{3-[N-(2,3-dihydroxypropyl)carbamoyl]-5-acétamido-2,4,6-triiodobenzoyl-N-(2,3-dihydroxypropyl)aminoacétyl-N-méthylamino}-2,4,6-triiodoisophtalamide.

25. N,N'-bis-(2,3-dihydroxypropyl)-N,N'-diméthyl-5-{3-[N-(2,3-dihydroxypropyl)carbamoyl]-5-hydroxyacétamido-2,4,6-triiodobenzoyl-N-méthylaminoacétyl-N-méthylamino}-2,4,6-triiodoisophtalamide.

26. N,N'-bis-(2,3-dihydroxypropyl)-N,N'-diméthyl-5-{3-[N-(2,3-dihydroxypropyl)carbamoyl]-5-méthoxyacétamido-2,4,6-triiodobenzoyl-N-méthylaminoacétyl-N-méthylamino}-2,4,6-triiodoisophtalamide.

27. N,N'-bis-(2,3-dihydroxypropyl)-N,N'-diméthyl-5-{3-[N-(2,3-dihydroxypropyl)carbamoyl]-5-hydroxyacétamido-2,4,6-triiodobenzoylaminoacétyl-N-méthylamino}-2,4,6-triiodoisophtalamide.

28. N,N'-bis-(2,3-dihydroxypropyl)-N,N'-diméthyl-5-{3-[N-(2,3-dihydroxypropyl)carbamoyl]-5-méthoxyacétamido-2,4,6-triiodobenzoylaminoacétyl-N-méthylamino}-2,4,6-triiodoisophtalamide.

29. N,N'-bis-(2,3-dihydroxypropyl)-5-{3-[N-(2,3-dihydroxypropyl)carbamoyl]-5-méthoxyacétamido-2,4,6-triiodobenzoylaminoacétyl-N-méthylamino}-2,4,6-triiodoisophtalamide.

30. N,N,N',N'-tétrakis-(2-hydroxyéthyl)-5-{3-[N-(2,3-dihydroxypropyl)carbamoyl]-5-hydroxyacétamido-2,4,6-triiodobenzoyl-N-méthylaminoacétyl-N-méthylamino}-2,4,6-triiodoisophtalamide.

31. N,N,N',N'-tétrakis-(2-hydroxyéthyl)-5-{3-[N-(2,3-dihydroxypropyl)carbamoyl]-5-méthoxyacétamido-2,4,6-triiodobenzoyl-N-méthylaminoacétyl-N-méthylamino}-2,4,6-triiodoisophtalamide.

32. N,N,N',N'-tétrakis-(2-hydroxyéthyl)-5-{3-[N-(2,3-dihydroxypropyl)carbamoyl]-5-méthoxyacétamido-2,4,6-triiodobenzoylaminoacétamido}-2,4,6-triiodoisophtalamide.

33. N,N'-bis-(2-hydroxyéthyl)-5-{3-[N-(2,3-dihydroxypropyl)carbamoyl]-5-méthoxyacétyl-N-(2-hydroxyéthyl)amino-2,4,6-triiodobenzoyl-N-(2-hydroxyéthyl)amineacétyl-N-(2-hydroxyéthyl)amino}-2,4,6-triiodoisophtalamide.

34. N,N'-bis-(2,3-dihydroxypropyl)-5-{3-[N-(2,3-dihydroxypropyl)carbamoyl]-5-méthoxyacétyl-N-(2-hydroxyéthyl)amino-2, 4, 6-triiodobenzoyl-N-(2-hydroxyéthyl)amineacétyl-N-(2-hydrexyéthyl)amino}-2,4,6-triiodoisophtalamide.

35. N,N'-bis-(2,3-dihydroxypropyl)-5-{3-[N-(2,3-dihydroxypropyl)carbamoyl]-5-hydroxyacétyl-N-(2-hydroxyéthyl}amino-2,4,6-triiodobenzoylaminoacétyl-N-(2-hydroxyéthyl)amino}-2,4,6-triiodoisophtalamide.

36. N,N'-bis-(2,3-dihydroxypropyl)-5-{3-[N-(2,3-dihydroxypropyl)carbamoyl]-5-méthoxyacétyl-N-(2-hydroxyéthyl)amino-2,4,6-triiodobenzoyl-N-méthylaminoacétyl-N-(2-hydroxyéthyl)amino}-2,4,6-triiodoisophtalamide.

37. N,N'-bis-(2,3-dihydroxypropyl)-5-{3-[N-(2,3-dihydroxypropyl)carbamoyl]-5-acétamido-2,4,6-triiodobenzoyl-N-(2,3-dihydroxypropyl)aminoacétamido}-2,4,6-triiodoisophtalamide.

38. N,N'-bis-(2,3-dihydroxypropyl)-5-{3-[N-(2,3-dihydroxypropyl)carbamoyl]-5-acétamido-2,4,6-triiodobenzoyl-N-méthylacétyl-N-méthylamino}-2,4,6-triiodoisophtalamide.

39. N,N'-bis-(1-hydroxyméthyl-2,3-dihydroxypropyl)-5-{3-[N-(2,3-dihydroxypropyl)carbamoyl]-5-acétamido-2,4,6-triiodobenzoyl-N-méthylacétyl-N-méthylamino}-2,4,6-triiodoisophtalamide.

40. N,N'-bis-(2,3-dihydroxypropyl)-5-{3-[N-(2,3-dihydroxypropyl)carbamoyl]-5-méthoxyacétamido-2,4,6-triiodobenzoyl-N-(2-hydroxy-3-méthoxypropyl)aminoacétyl-N-méthylamino}-2,4,6-triiodoisophtalamide.

41. N,N'-bis-(2,3-dihydroxypropyl)-5-{3-[N-(2,3-dihydroxypropyl)carbamoyl]-5-hydroxyacétyl-N-(2-méthoxyéthyl)amino-2,4,6-triiodobenzoylaminoacétyl-N-(2-méthoxyéthyl)amino}-2,4,6-triiodoisophtalamide.

42. N,N'-bis-(2,3-dihydroxypropyl)-5-{3-[N-(2-hydroxyéthyl)-N-(2,3-dihydroxypropyl)carbamoyl]-5-méthoxyacétamido-2,4,6-triiodobenzoyl-N-méthylaminoacétyl-N-méthylamino}-2,4,6-triiodoisophtalamide.

43. N,N'-bis-(2-hydroxyéthyl)-N,N'-bis-(2,3-dihydroxypropyl)-5-{3-[N-(2,3-dihydroxypropyl)carbamoyl]-5-acétamido-2,4,6-triiodobenzoyl-N-méthylaminoacétyl-N-méthylamino}-2,4,6-triiodoisophtalamide.

44. Compositions galéniques qui comprennent le composé de formule générale I, définie dans la revendication 1, en des concentrations entre 100 et 400 mg de I par mL, et incorporant des stabilisateurs de pH tels que le tampon Tris (= tris(hydroxyméthyl)aminométhane) et ses sels, phosphates, citrates, et hydrogénocarbonates, ainsi que de l'eau stérile apyrogène, et peuvent aussi incorporer des agents complexants pour métaux lourds, par exemple, les sels de sodium et/ou de calcium de l'acide éthylène-diaminetétraacétique, et d'autres agents chélatants qui sont pharmaceutiquement acceptables.

45. Procédés de préparation des composés de formule générale I, définie dans la revendication 1, caractérisés en ce qu'ils sont formés par réaction d'un composé de formule générale II : 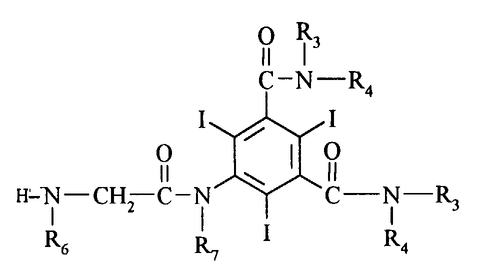 avec un chlorure d'acide de formule III : 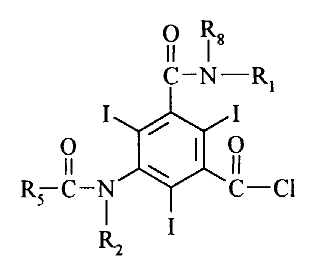 en présence d'un catalyseur basique, les radicaux R₁ à R₈ ayant la signification indiquée précédemment; durant la réaction, les groupes -OH sont protégés en tant qu'acétates ou, lorsque les groupes -OH sont voisins, en tant que dioxolanes.

46. Procédé de préparation des composés de formule générale I au moyen d'une réaction de N-alkylation utilisant les réactifs chlorure de 2-hydroxyéthyle, de 2-méthoxyéthyle, d'hydroxy-3-méthoxypropyle et de 2,3-dihydroxypropyle en milieu alcalin, avec les composés de formule I dans lesquels R₂ et R₇ sont l'hydrogène.
